(19) Europäisches Patentamt
European Patent Office
Office européen des brevets

(11) Publication number: 0 186 275 A2

(12) # EUROPEAN PATENT APPLICATION

(21) Application number: 85307614.9

(22) Date of filing: 22.10.85

(51) Int. Cl.⁴: C 07 D 239/46
C 07 D 401/12, A 61 K 31/505

(30) Priority: 28.12.84 JP 277760/84

(43) Date of publication of application: 02.07.86 Bulletin 86/27

(84) Designated Contracting States: AT BE CH DE FR GB IT LI NL SE

(71) Applicant: YAMANOUCHI PHARMACEUTICAL CO. LTD.
No. 5-1 Nihonbashi-Honcho, 2-chome Chuo-ku
Tokyo(JP)

(72) Inventor: Yanagisawa, Isao
No. 22-8, Shakujiidai 2-chome Nerima-ku
Tokyo(JP)

(72) Inventor: Ohta, Mitsuaki
No. 18-20, Minami 1-chome
Wako-shi Saitama(JP)

(72) Inventor: Takagi, Tokuichi
No. 27-13, Takasago 8-chome Katsushika-ku
Tokyo(JP)

(72) Inventor: Takeuchi, Makoto Yamanouchi Seiyaku
Hasune ryo No.16-1, Hasune 3-chome
Itabashi-ku Tokyo(JP)

(74) Representative: Geering, Keith Edwin et al,
REDDIE & GROSE 16 Theobalds Road
London WC1X 8PL(GB)

(54) **2-Substituted amino-4(1H)-pyrimidone derivatives, their production, and medical compositions containing them.**

(57) A 2-substituted amino-4 (1H)-pyrimidone derivative shown by the general formula

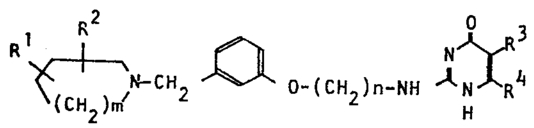

wherein $R^1$ and $R^2$, which may be the same or different, each represents a hydrogen atom or a lower alkyl group; $R^3$ represents an alkyl group, an alkenyl group, an alkynyl group, a halogen-substituted alkyl group, a halogen-substituted alkenyl group, a halogen-substituted alkynyl group, or an alkyl group interrupted by an oxygen atom or a sulfur atom; $R^4$ represents a hydrogen atom or a lower alkyl group; m is an integer of 1 or 3 and n is an integer of 2 or 4 (with said $R^4$ being a lower alkyl group when $R^1$ and $R^2$ are hydrogen atoms, $R^3$ is an alkyl group, m is 2 and n is 3) or an acid addition salt thereof.

The compounds of this invention have both histamine $H^2$-receptor antagonist activity and gastric cytoprotective activity and are very useful as drugs for gastric diseases.

# 2-SUBSTITUTED AMINO-4(1H)-PYRIMIDONE DERIVATIVES, THEIR PRODUCTION, AND MEDICAL COMPOSITIONS CONTAINING THEM

This invention relates to 2-substituted amino-4(1H)-pyrimidone derivatives useful as drugs for gastric diseases, production of these compounds, and medical compositions containing them.

There are known histamine $H_1$-receptors inhibited by medicaments generally called "antihistamines" such as mepyramine, diphenhydramine, etc., and histamine $H_2$-receptors not inhibited by such "antihistamines" but inhibited by medicaments such as burimamide, cimetidine, etc., (Ash & Shild, "Brit. J. Pharmacol. Chemother."; 27, 427(1966) and Black et al, "Nature"; 236, 385(1972)).

The compounds antagonizing these receptors are

called histamine $H_1$-antagonists and histamine $H_2$-antagonists respectively.

A histamine $H_2$-antagonist is effective for the treatment of diseases caused by the physiological action of histamine in which a histamine $H_2$-receptor intervenes, for example, for the treatment of gastric ulcer caused by hypersecretion of gastric acid, for the treatment of duodenal ulcer, and for inhibition of gastric acid secretion.

Also, for the prophylaxis and the treatment of gastric diseases, there are other medicaments which act to increase the normal protective function of the body

and these medicaments are called "gastric cytoprotective drugs" (cytoprotective drugs). As such medicaments, prostaglandin, etc., are known.

This invention provides 2-substituted amino 4(1H)-pyrimidone derivatives represented by following general formula (I) and acid addition salts thereof

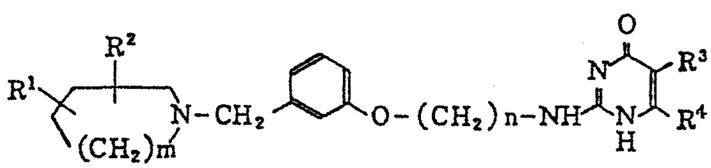 (I)

wherein $R^1$ and $R^2$ are the same or different and selected from a hydrogen atom and lower alkyl groups; $R^3$ represents an alkyl group, an alkenyl group, an alkynyl group, an alkyl group substituted by halogen,

an alkenyl group substituted by halogen, an alkynyl group substituted by halogen, or an alkyl group interrupted by an oxygen atom or a sulfur atom; $R^4$ represents a hydrogen atom or a lower alkyl group; m represents an integer of 1 to 3; and n represents an integer of 2 to 4, said $R^4$ being, however, a lower alkyl group when $R^1$ and $R^2$ are a hydrogen atom, $R^3$ is an alkyl group, m is 2 and n is 3.

The compounds of this invention have both histamine $H_2$-receptor antagonist activity and gastric cytoprotective activity and hence are very useful as drugs for gastric diseases, such as antiulcer drugs, gastric acid secretion inhibitors, etc.

UK 2030979.A discloses compounds of foregoing general formula (I) wherein both $R^1$ and $R^2$ are hydrogen atoms, $R^3$ is an alkyl group, $R^4$ is a hydrogen atom, m is 2, and n is 3, but these compounds are different from those of this invention. Also, compounds of general formula (I) wherein both $R^1$ and $R^2$ are hydrogen atoms, $R^3$ is an alkyl group, $R^4$ is a lower alkyl group, m is 1 or 2, and n is 3 are theoretically claimed but not explicitly and practically disclosed in European Patent No. 13,071. On the other hand, the

compounds of this invention are very useful in having both histamine $H_2$-receptor antagonist activity and gastric cytoprotective activity.

The term "lower alkyl group" herein

means a straight chain or branched alkyl group having 1 to 5 carbon atoms and specific examples are a methyl group, an ethyl group, a propyl group, an isopropyl group, a butyl group, an isobutyl group, a sec-butyl group, a tert-butyl group, a pentyl (amyl) group, an isopentyl group, etc. The other alkyl groups herein are also straight or branched chain, having 1 to 10 carbon atoms. Specific examples of the alkyl group $R^3$ are the above-described lower alkyl groups and further a hexyl group, an isohexyl group, a 1-methylpentyl group, a 2-ethylbutyl group, a heptyl group, a 5-methylhexyl group, a 1-methylhexyl group, a 3-ethylpentyl group, a 2-propylbutyl group, an octyl group, a 6-methylheptyl group, a nonyl group, a 7-methyloctyl group, a decyl group, an 8-methylnonyl group, etc.

The alkenyl and alkynyl groups herein have unsaturated straight or branched $C_2$ to $C_{10}$ carbon chains, preferably having a double bond or a triple bond, respectively, at

one or two positions in the carbon chain.

Specific examples of the alkenyl and alkynyl groups are a vinyl group, an allyl group, a 2-butenyl group ($CH_3-CH=CH-CH_2-$), a 3-butenyl group, a 1,3-butanedienyl group, a 2-pentenyl group, an isopropenyl group ($CH_2=\underset{CH_3}{C}-$ ), a 3-methyl-2-butenyl group ($CH_3-\underset{CH_3}{C}=CH-CH_2-$), an ethynyl group, a 2-propynyl group, a 3-pentynyl group, a 2-methyl-3-butynyl group, étc.

The alkyl, alkenyl and alkynyl groups substituted by halogen may have one or more than one halogen substituent at any position(s); examples are a trifluoromethyl group, a 2-chloroethyl group, a 3-bromopropyl group, a 3,3,3,-trichloropropyl group, a 2,2,2,-trifluoroethyl group, a 3,4,4,-trifluoro-3-butenyl group, a 2,3,3,-trifluoropropenyl group, etc.

The term "alkyl group interrupted by an oxygen atom or a sulfur atom" means herein an alkyl group of 1 to 10 carbon atoms, in which the carbon chain is interrupted by an oxygen atom or a sulfur atom and specific examples are a 3-methoxypropyl group, a 2-ethoxyethyl group, a 3-methylthiopropyl group, a 2-ethylthioethyl group, a 2-isopropylthioethyl group, a 2-(2-methylpropylthio)ethyl group, etc.

The term "lower alkoxy group" in general formula (III) below

means an alkoxy group having 1-5 carbon atoms such as a methoxy group, ethoxy group, propoxy group, butoxy group, pentyloxy group, etc. a "lower alkylthio group" likewise has 1 to 5 carbon atoms, and examples of the "aralkylthio group" are the benzylthio group, benzhydrylthio group, tritylthio group, etc.

As the acid addition salts of the compounds shown by general formula (I), there are mineral acid salts such as hydrochlorides, hydrobromides, hydroiodides, sulfates, nitrates, phosphates, etc., and acid addition salts with organic acids, such as fumarates, maleates, picrates, etc.

Compounds of this invention can exist as 1H and 3H tautomers or as hydroxy tautomers. When the $R^3$ substituent on the pyrimidone ring has a double bond, this results in cis-trans isomers (geometrical isomers). Furthermore, when at least one of $R^1$ and $R^2$ is lower alkyl, then according to their positions on the ring one or two of the ring carbon atoms may be assymmetric so that optical isomers exist. All of the isomers, i.e. the tautomers, the geometrical isomers, the optical isomers, etc., individually and in any combination, are included in this invention.

The compounds of this invention shown by general formula (I) and the acid addition salts thereof can be produced by various processes. Typical production processes of the compounds of this invention are illustrated below.

Production process A:

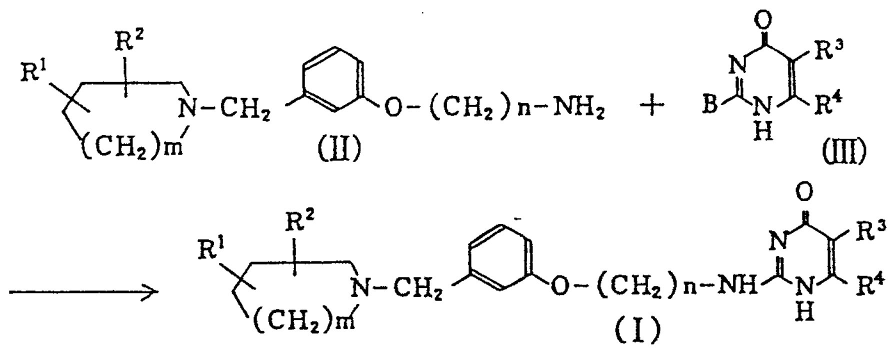

(II) (III) (I)

wherein, $R^1$, $R^2$, $R^3$, $R^4$, m and n are as defined above and B represents a nitroamino group, a lower alkylthio group, a lower alkoxy group or an aralkylthio group; e.g. a compound of this invention shown by general formula (I) is produced by reacting a phenoxyalkylamine shown by general formula (II) and a 2-nitroamino- or 2-lower alkylthio-4(1H)-pyrimidone derivative shown by general formula (III).

This reaction can be performed without using a solvent, in which case it is advantageous to perform the reaction at a high temperature by heating. The reaction can also be performed

in an organic solvent inactive to the reaction (such as pyridine, acetonitrile, toluene, dimethoxyethane, methanol and ethanol) with heating, preferably under reflux. It is preferred that raw materials shown by formulae (II) and (III) be used in equimolar amounts or with one raw material in slight excess.

When a salt of the compound of formula (II) is used as the raw material and the salt of the compound is reacted with the compound of formula (III) wherein B is a lower alkylthio group in an organic solvent, if circumstances require, it is preferred that the reaction be performed with the addition of a base such as sodium ethoxide.

The reaction time varies according to the reaction conditions employed.

Production process B:

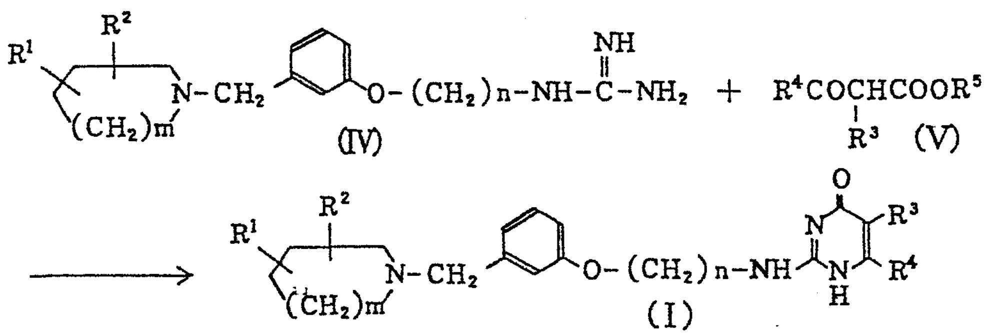

wherein, $R^1$, $R^2$, $R^3$, $R^4$, m and n are as defined above and $R^5$ represents a lower alkyl group.

A compound of this invention shown by general formula (I) can be also produced by the condensation cyclization reaction of a phenoxyalkylguanidine shown by general formula (IV) and an acylacetic acid ester

derivative shown by general formula (V).

It is advantageous to perform the reaction using almost equimolar amounts of the compounds of formulae (IV) and (V) or with one of these in a slight excess, in an organic solvent inactive to the reaction - for example, an alcohol such as methanol, ethanol, isopropanol, etc., dimethylformamide, dimethyl sulfoxide, methylcellosolve (methoxy ethanol), ethylcellosolve (ethoxy ethanol), digrim [bis(2-methoxyethyl) ether], dioxane, etc., at room temperature or under heating. In the reaction, a base may be added if necessary and as such a base, an alkali metal alcoholate such as sodium methoxide, sodium ethoxide, etc., or potassium hydroxide, sodium hydroxide, etc., is preferably used.

Production process C:

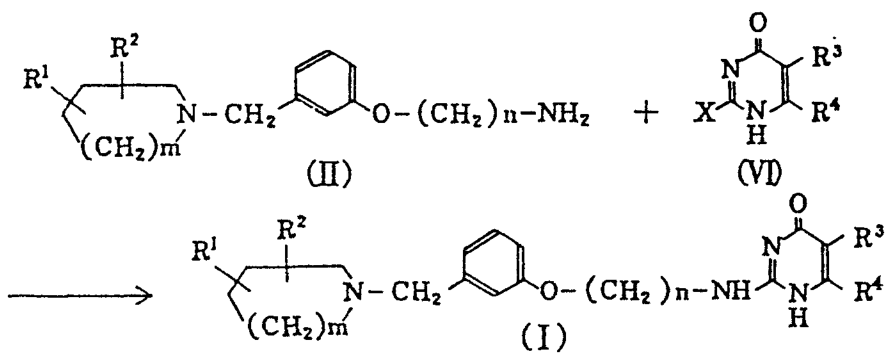
(II) (VI) (I)

wherein, $R^1$, $R^2$, $R^3$, $R^4$, m and n are as defined above and X represents a halogen atom.

A compound of general formula (I) can be further produced by reacting a compound of formula (II) as a raw material and a 2-halogeno-4-(1H)-pyrimidone

derivative shown by general formula (VI).

Practical examples of the halogen atom shown by X are iodine atom, bromine atom, chlorine atom, etc.

The reaction proceeds without solvent but is preferably performed in an organic solvent inactive to the reaction. Examples of the organic solvent are benzene, toluene, xylene, dimethylformamide, dichloromethane, dichloroethane, methanol, ethanol, etc.

It is sometimes advantageous for smooth reaction to add a tertiary base such as pyridine, picoline, N,N-dimethylaniline, triethylamine, etc., or a base such as potassium carbonate, sodium carbonate, sodium hydrogencarbonate, etc., to the reaction. In this case, pyridine may sometimes be used as the solvent for performing the reaction.

Production process D:

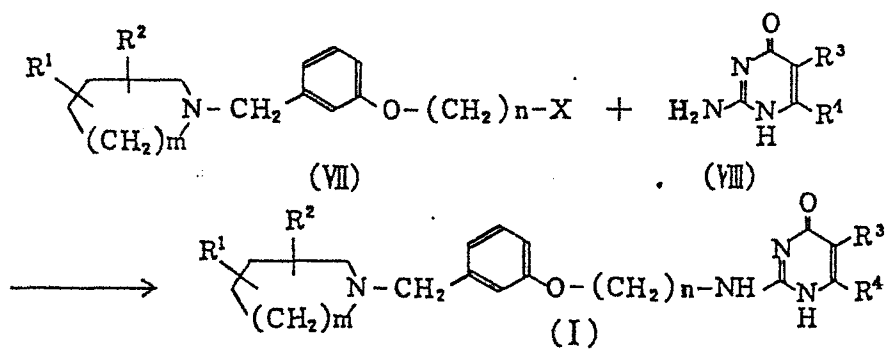

wherein $R^1$, $R^2$, $R^3$, $R^4$, X, m and n are as defined above.

A compound of general formula (I) can be also produced by reacting a halogen compound shown by general

formula (VII) and a 2-amino-4(1H)-pyrimidone derivative shown by general formula (VIII).

The reaction can be practiced under the same reaction conditions as Production process C.

Production process E:

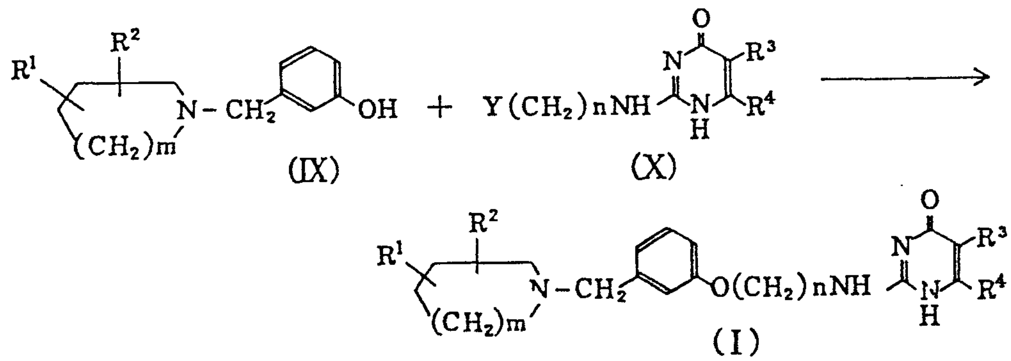

(IX) (X) (I)

wherein, $R^1$, $R^2$, $R^3$, $R^4$, m and n are as defined above and Y represents a halogen atom, or an organo-sulfonyloxy group such as a methanesulfonyloxy group, a p-toluenesulfonyloxy group, etc.

A compound of this invention shown by general formula (I) can be also produced by reacting a phenol derivative shown by general formula (IX) and a pyrimidone derivative shown by general formula (X) in the presence of an alkali.

Suitable examples of the alkali are an alkali metal alcoholate such as sodium methoxide, sodium ethoxide, etc., or sodium hydride, sodium hydroxide, potassium hydroxide, etc.

The reaction is performed in a solvent such as methanol, ethanol, dimethylformaide, dimethyl sulfoxide, etc. It is preferred to perform the reaction at room temperature or under heating.

The compounds of this invention thus obtained can be converted into the acid addition salts thereof by subjecting the compounds to ordinary salt forming reactions.

The compounds of this invention thus formed can be isolated and purified by conventional chemical operations such as solvent distillation, crystallization, column chromatography, recyrstallization, etc.

Since a compound of general formula (I) (or the acid addition salt thereof) provided by this invention has both histamine $H_2$-receptor antagonist activity and gastric cytoprotective activity (cytoprotective activity), these compounds are useful as gastric acid secretion inhibitors, drugs for grastric ulcer, etc., showing less side reaction.

These effects of the compounds of this invention have been confirmed by the following experiments.

In addition, the experiments were performed using a known compound, TZU-0460, i. e., N-[3-[3-(1-piperidinylmethyl)phenoxy]propyl]acetoxyacetamide hydrochloride shown by the following formula

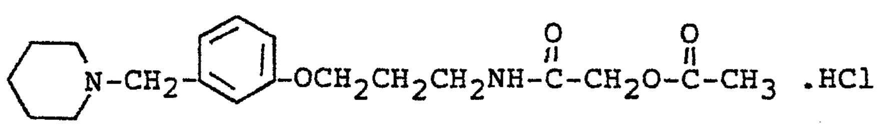

disclosed in European Patent No. 24,510.

1. Experimental procedure

1) $H_2$-Blocking activity:

The atrium isolated from quinea-pigs was mounted in an organ bath containing Krebs-Henseleit solution

gassed with 95% $O_2$ and 5% $CO_2$ at 37°C. The spontaneous movement of the atrium was recorded through a strain gauge (Nihon Koden, SB-1T) on a polygraph (Nihon Koden, RM-6200). The tissue was exposed to test drugs 30 min. before treatment with 5 x $10^{-6}$M histamine. The dose of test drugs producing 50%blockade of chronotropic response to histamine was obtained from each preparation.

2) Gastric secretion inhibiting activity:

Male Wistar rats (about 200 g) were fasted but allowed free access to water for 24 hours prior to the experiment. The pylorus was ligated after abdominal midline incision under ether anesthesia. Four hours later, the animals were killed for the collection of gastric juice. The test compounds were given p.o. 1 hour before the pylorus ligation.

Gastric juice (0.5 ml) was titrated against 0.05 N NaOH up to pH 7.0 to determine acid concentration using an automatic titrator (Hiranuma Sangyo Co., Comtite 7). The total acid output was calculated as the product of gastric volume by acid concentration.

3) Gastric cytoprotection:

Male Wistar rats (about 200 g) were starved for 24 hours and deprived of water for 18 hours prior to the experiment.

Absolute ethanol (1 ml/rat) was given orally to rats one hour after p.o. administration of the test compounds. One hour later, animals were sacrificed for the measure-

ment of gastric lesions ($mm^2$).

2. Test result

| Example No. | 1) $H_2$-blockade | 2) Anti-secretion | 3) Cytoprotection | $LD_{50}$ (mg/kg iv) |
|---|---|---|---|---|
| | guinea pig atrium $ED_{50}$ (M) | 4hr. shay rat $ED_{50}$ (mg/kg p.o.) | ethanol ulcer rat $ED_{50}$ (mg/kg p.o.) | |
| 1 | $>10^{-6}$ | 2.6 | 6.0 | – |
| 4 | $4.2x10^{-7}$ | 6.4 | 5.8 | 61.1 |
| 5 | $8.9x10^{-6}$ | 19.0 | 9.6 | – |
| 7 | $5.1x10^{-7}$ | 6.1 | 6.6 | – |
| 9 | $2.1x10^{-7}$ | 3.1 | 23.3 | – |
| 12 | $6.1x10^{-7}$ | 16.4 | 6.7 | 42.5 |
| 14 | $8.2x10^{-7}$ | 9.9 | 41.4 | – |
| 15 | $4.0x10^{-7}$ | 10.9 | 25.4 | – |
| 26 | $3.6x10^{-7}$ | 5.2 | 20.5 | – |
| 30 | $1.7x10^{-7}$ | 18.0 | 17.0 | – |
| 31 | $2.9x10^{-7}$ | 30.1 | 13.6 | – |
| TZU-0460 | $3.7x10^{-7}$ | 35.1 | 46.4 | – |

Formulations containing compounds shown by general formula (I) above or acid addition salts thereof can be prepared in conventional manner using conventional optional carriers and excipients.

The compounds of the invention may be administered orally or parenterally. In the case of oral administration, the compound can be formulated into syrup, tablet, capsule, etc. form. The syrup is composed of a suspension or solution of the compound, a sweetening agent, etc., in a liquid carrier such as ethanol, glycerol, and water. In the case of tablets, a carrier usually used for solid formulations, such as starch, lactose, sucrose, cellulose, etc., can be used.

In the case of parenteral administration, the medicament is composed of a solution or suspension of the compound of formula (I) or an acid addition salt thereof in a sterilized water carrier or a parenterally acceptable oil.

A preferred medical composition of this invention is a unit dosing agent form such as a tablet or capsule, which can be administered by a patient by himself.

The doses are determined according to the symptom, age, sex, etc., of the patient but the total is usually 30 to 400 mg per adult per day, administered

in a single dose or in 2 to 4 doses.

The invention is illustrated in more detail by the following Reference Examples and Examples.

Reference Example 1

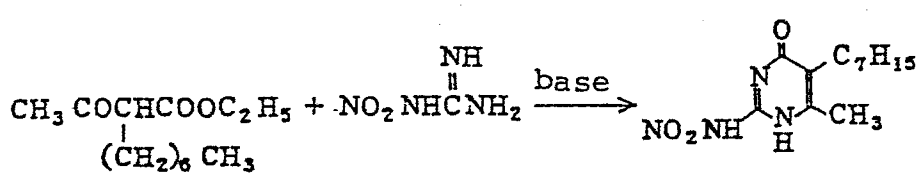


In 35 ml of absolute methanol was dissolved 0.80 g of metallic sodium ;after adding 3.2 g of nitroguanidine to the solution, the mixture was stirred for 45 minutes under reflux. Then 6.1 g of ethyl 2-heptyl-3-oxobutanoate was added to the mixture and the resultant mixture was stirred overnight under reflux. Then, after distilling off the solvent under reduced pressure, 100 ml of water was added to the residue. The solution thus formed was acidified by the addition of concentrated hydrochloric acid to deposit crystals, which were collected by filtration and recrystallized from ethanol to provide 4.61 g of 5-heptyl-6-methyl-2-nitroamino-4(1H)-pyrimidone showing a melting point of 159.5 to 160.5°C.

Mass spectrum (EI)

m/z: 268 ($M^+$)

By following almost the same procedure as in Reference Example 1, the following compounds were obtained :

Reference Example 2

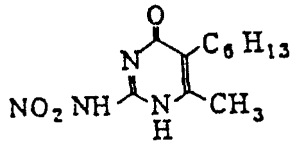

5-Hexyl-6-methyl-2-nitroamino-4(1H)-pyrimidone

m.p. 161.0 - 162.0°C Mass spectrum(EI) m/z : 254($M^+$)

Reference Example 3

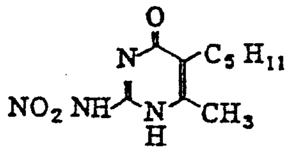

6-Methyl-2-nitroamino-5-pentyl-4(1H)-pyrimidone
m.p. 164.0 - 165.0°C
Mass spectrum(EI) m/z :240($M^+$)

Reference Example 4

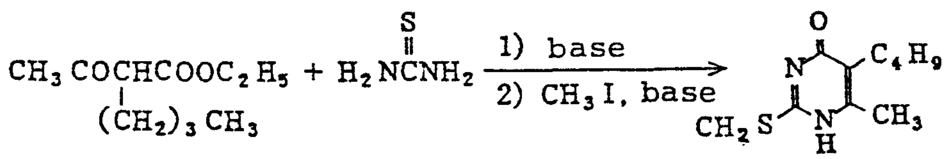


In 20 ml of absolute ethanol was dissolved 0.58 g of metallic sodium and after adding thereto 4.66 g of ethyl 2-butyl-3-oxobutanoate and then 1.9 g of thiourea, the mixture was stirred for one hour under reflux. Then, after distilling off the solvent under reduced pressure, 20 ml of water was added to the residue thus formed and the solution thus formed was acidified with concentrated hydrochloric acid to deposit crystals, which were collected by filtration and recrystallized from ethanol. Then. 2.52 g of the

crystals thus obtained, 1.85 g of methyl iodide, and 1.05 g of sodium hydroxide were added to 40 ml of water and the mixture was stirred for 1.5 hours at 70°C and then overnight at room temperature. The solution thus formed was acidified with concentrated hydrochloric acid to deposit crystals, which were collected by filtration and washed with water to provide 2.47 g of 5-butyl-6-methyl-2-methylthio-4(1H)-pyrimidone showing a melting point of 154.0 to 155.0°C.

Mass spectrum (EI)

m/z: 212 ($M^+$)

Reference Example 5

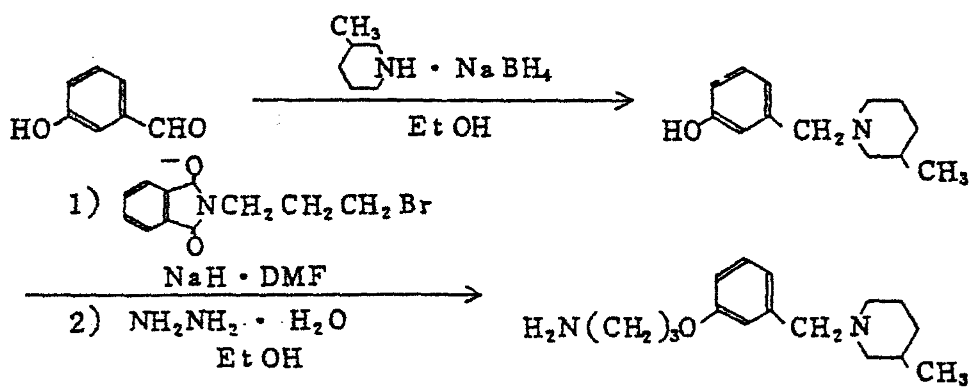


3-[m-(3-Methylpiperidinomethyl)phenoxy]propylamine:

(a) To 250 ml of absolute ethanol were added 20.0 g of m-hydroxybenzaldehyde and 42.0 g of 3-methylpiperidine and the mixture was cooled below 5°C by an ice-water bath followed by stirring. After 20 minutes, 6.2 g of sodium borohydride was added to the solution and after stirring the mixture for 21 hours at room temperature, the solvent was distilled off

under reduced pressure. Then, 300 ml of 10% hydrochloric acid was added to the residue thus formed to provide an acid aqueous solution. The aqueous solution was washed thrice each time with 100 ml of ethyl acetate and then alkalified by passing therethrough ammonia gas under ice-cooling to deposit crystals, which were collected by filtration, washed with water, and dried to provide 28.7 g of m-(3-methylpiperidinomethyl)phenol showing a melting point of 150 to 160°C.

Mass spectrum :

(m/z): 204 ($M^+$-1)

(b) In 250 ml of absolute N,N-dimethylformamide was dissolved 27.0 g of m-(3-methylpiperidinomethyl)-phenol thus obtained, then 5.8 g of sodium hydride was added to the solution under ice-cooling, and after stirring the mixture for 20 minutes at room temperature, 36.0 g of N-(3-bromopropyl)phthalimide was added to the mixture followed by stirring for 16 hours at 70°C. Then, the solvent was distilled off from the reaction mixture under reduced pressure, 350 ml of water was added to the residue thus formed, and the product was extracted with ethyl acetate. The ethyl acetate layer was washed with water and after drying with anhydrous magnesium sulfate, the solvent was distilled off under reduced pressure. After adding 200 ml of ethanol to the residue thus formed to form a homogeneous solution, 4.3 g of hydrazine (hydrate) was added to the solution and the mixture was refluxed for 9 hours.

Insoluble matters were filtered off, the solvent was distilled off under reduced pressure, and the residue thus formed was purified by column chromatography using chloroform-methanol-ammonia as a development solvent to provide 14.0 g of the oily desired product.

Nuclear magnetic resonance spectra (in $CDCl_3$)

$\delta$(ppm): 0.81 (3H, d)

140-2.25 (11H, m)

2.72-2.97 (4H, m)

3.43 (2H, s)

4.04 (2H, t)

6.70-7.30 (4H, m)

Mass spectra:

(m/z): 262 ($M^+$), 219, 188

By following the same procedure as Reference Example 5, the following raw material compounds were obtained.

Reference Example 6

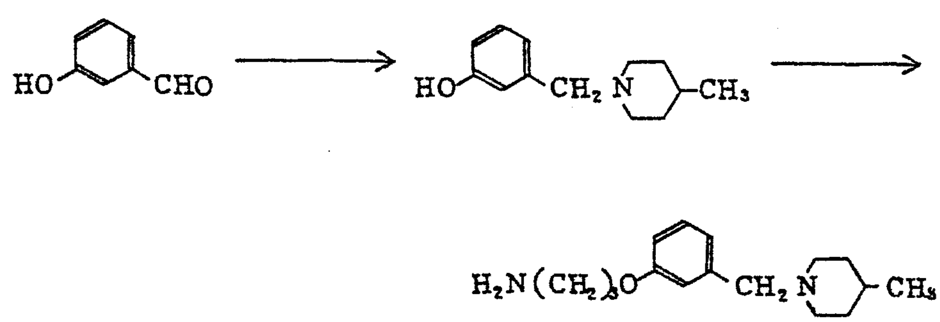

(a) m-(4-Methylpiperidinomethyl)phenol

Mass spectrum: (m/z) 204($M^+$-1)

(b) 3-[m-(4-Methylpiperidinomethyl)phenoxy]-propylamine

NMR spectra (in $CDCl_3$)

δ(ppm): 0.89 (3H, d)

1.04 - 2.03 (11H, m)

2.70 - 2.96 (4H, m)

3,43 (2H, s)

4.06 (2H, t)

6.68 - 7.31 (4H, m)

Mass spectra: (m/z) 261 ($M^+$-1), 204, 165

Reference Example 7

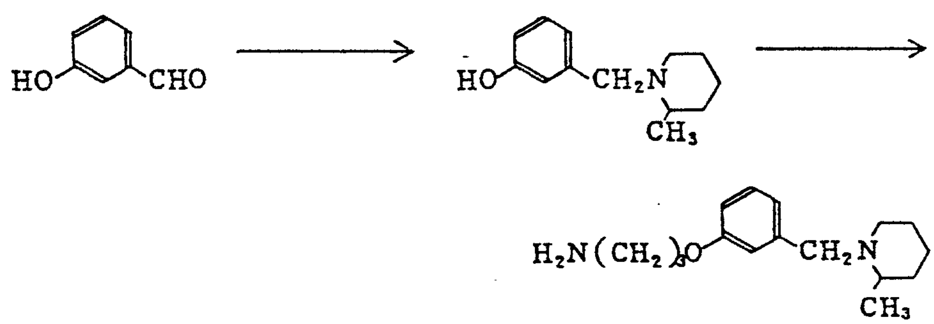

(a) m- (2-Methylpiperidinomethyl)phenol

Mass spectrum: (m/z) 205 ($M^+$)

(b) 3-[m-(2-Methylpiperidinomethyl)phenoxy]-propylamine

NMR spectra (in $CDCl_3$)

δ(ppm): 1.14 (3H, d)

1.20 - 2.11 (11H, m)

2.28 (1H, m)

2.60 - 3.22 (4H, m)

3.88 - 4.12 (3H, m)

6.64 - 7.32 (4H, m)

Mass spectra: (m/z) 261 ($M^+$-1), 247, 190

Reference Example 8

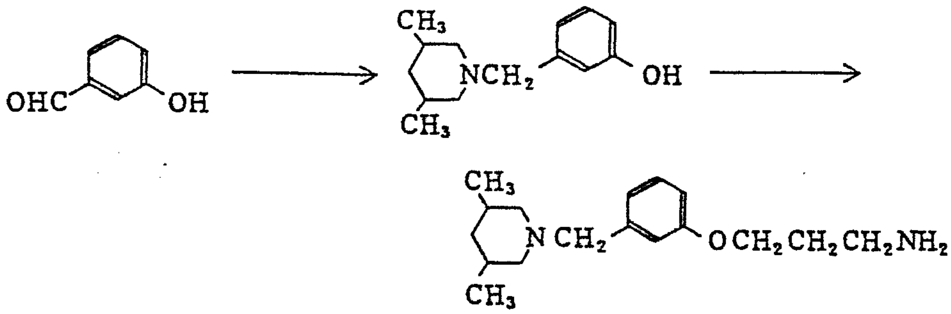

(a) m-(3,5-Dimethylpiperidinomethyl)phenol

Mass spectrum: (m/z) 219 ($M^+$)

(b) 3-[m-(3,5-Dimethylpiperidinomethyl)phenoxy]-propylamine

NMR spectra (in DMSO-$d_6$)

δ(ppm); 0.76 (3H, d)

0.90 (3H, d)

1.12 - 2.12 (8H, m)

2.20 - 3.10 (6H, m)

3.36 (2H, s)

3.98 (2H, s)

6.68 - 7.26 (4H, m)

Mass spectra: (m/z) 276 ($M^+$), 165

Reference Example 9

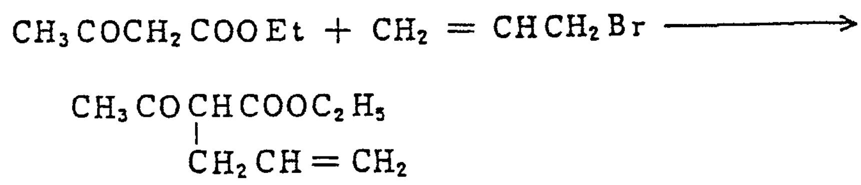

In 100 ml of absolute ethanol was dissolved 2.14 g of metallic sodium under nitrgen stream and 12.1 g of ethyl acetoacetate and then 11.3 g of allyl bromide were added to the solution. The mixture was stirred for one day at room temperature and then for 6 hours under reflux and then the solvent was distilled off under reduced pressure. To the residue thus formed was added 100 ml of water and the product was extracted with ethyl acetate (100 ml x 2). The organic layer thus formed was collected and after drying with magnesium sulfate, the solvent was distilled off under reduced pressure. The oily residue thus formed was distilled under reduced pressure to provide 9.3 g of the desired product.

Boiling point: 110-113°C (35 mmHg)

Mass spectrum:

(m/z): 171 ($M^+$ + 1)

By following the same procedure as Reference Example 9, the following compounds were prepared.

Reference Example 10

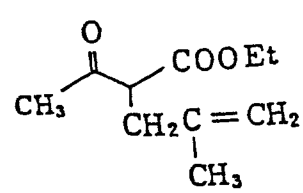

b.p. 110 - 112°C (22 mmHg)

Mass spectrum: (m/z) 185 ($M^{+}$+1)

Reference Example 11

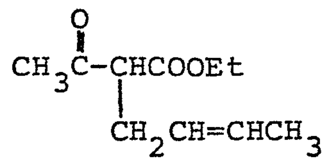

b.p. 60 - 61°C (0.3 - 0.4 mmHg)

Mass spectrum: (m/z) 185 ($M^{+}$+1)

Reference Example 12

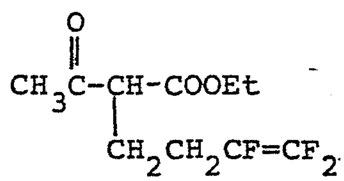

b.p. 103 - 107°C (12 mmHg)

Reference Example 13

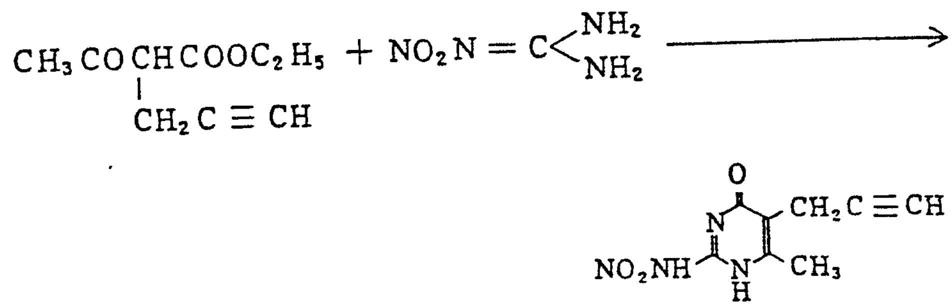

In 60 ml of absolute methanol was dissolved 0.68 g of metallic sodium and after adding thereto 3.09 g of nitroguanidine, the mixture was refluxed for one hour. The reaction mixture was allowed to cool to room temperature and after adding thereto a solution of 5.0 g of ethyl (α-propynyl)acetoacetate dissolved in 30 ml of methanol, the mixture was refluxed for 20 hours.

The solvent was distilled off under reduced pressure and after dissolving the residue thus formed in 100 ml of water, the solution was extracted with ether (50 ml x 3). The aqueous layer thus formed was acidified with concentrated hydrochloric acid under ice-cooling and crystals thus formed were collected by filtration to provide 2.93 g of 2-nitroamino-5-propynyl-6-methyl-4-pyrimidone showing a boiling point higher than 300°C.

Mass spectra:

(m/z): 208 ($M^+$), 162

By following the same procedure as Reference Example 13, the following compounds were obtained:

Reference Example 14

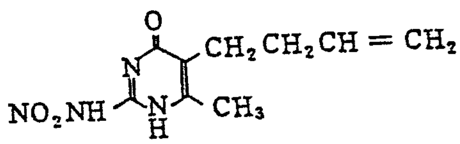

m.p. 139 - 140°C

Mass spectra: (m/z) 224 ($M^+$), 183

Reference Example 15

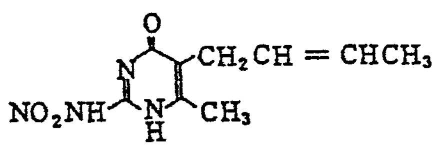

m.p. 162 - 164°C (decomposition)

Mass spectra: (m/z) 224($M^+$), 162

Reference Example 16

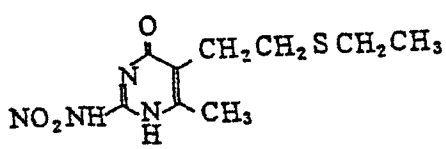

m.p. 184 - 186°C (decomposition)

Mass spectrum: (m/z) 258 ($M^+$)

Reference Example 17

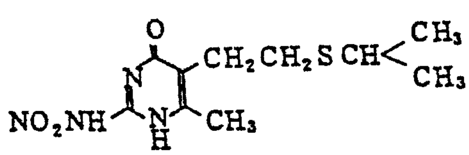

m.p. 186 - 187°C (decomposition)

Mass spectrum: (m/z) 272 ($M^+$)

Reference Example 18

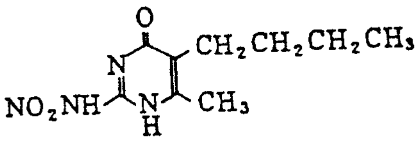

m.p. 163 - 164°C

Mass spectrum: (m/z) 226 ($M^+$)

Reference Example 19

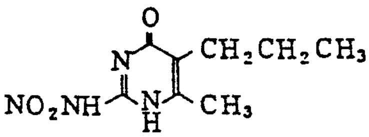

m.p. 166-167°C (recrystallized from ethanol)

Mass spectrum: (m/z) 212 ($M^+$)

Reference Example 20

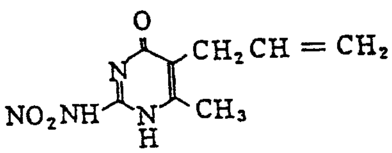

m.p. 158 - 159°C (recrystallized from ethanol)

Mass spectrum: (m/z) 210 ($M^+$)

Reference Example 21

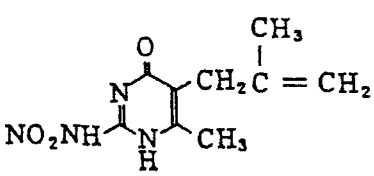

m.p. 183 - 185°C (recrystallized from ethanol)

Mass spectrum: (m/z) 224 ($M^+$)

Reference Example 22

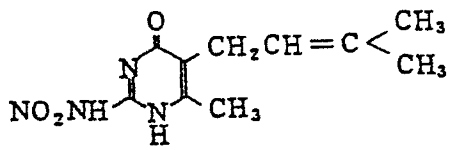

m.p. 185 - 186°C (recrystallized from ethanol)

Mass spectrum: (m/z) 238 ($M^+$)

Reference Example 23

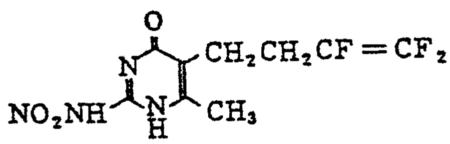

m.p. 172 - 173°C

Mass spectrum: (m/z) 278 ($M^+$)

Reference Example 24

Synthesis of ethyl 2-(2-(E)-butenyl)-3-oxo-butanoate

$$\begin{array}{l} CH_3CO\underset{|}{C}HCOOC_2H_5 \\ \quad\quad\;\; CH_2\underset{E}{CH{=}CH}CH_3 \end{array}$$

In 290 ml of ethanol was dissolved 11.1 g of metallic sodium under nitrogen gas stream. To the solution were added a solution of 62.5 g of ethyl 3-oxo-butanoate dissolved in 290 ml of toluene and then a solution of 56.0 g of 1-chloro-2(E)-butene [synthesized by the R.M. Magid et al method (Tetrahedron Letters, 1977, 2999)] dissolved in 290 ml of toluene and after stirring the resultant mixture for a whole day and night at room temperature, the mixture was further stirred for 3.5 hours under reflux. The solvents were distilled off under reduced pressure, water was added to the residue thus formed, and the mixture was rendered weakly acidic with an aqueous ammonium chloride solution. The product was extracted with methylene chloride, the extract was dried by anhydrous magnesium sulfate and distilled to provide 46.5 g of the desired product.

Boiling point: 100 to 105$^{o}$C/13 mm Hg

Mass spectrum (m/z): 185 ($M^+$ + 1)

(It was confirmed by $^{13}C$ NMR that trans/cis was 96.3/3.7)

Reference Example 25

Synthesis of 5-(2-(E)-butenyl)-6-methyl-2-nitroamino-4-(1H)-pyrimidone

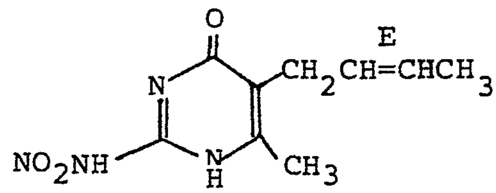

To 200 ml of methanol were added 104 ml of a methanol solution of 28% sodium methoxide and then 30.4 g of nitroguanidine and the mixture thus obtained was stirred for 45 minutes under reflux. After cooling the mixture, a solution of 51 g of 2-(2-(E)-butenyl)-3-oxo butanoate dissolved in 50 ml of methanol was added to the mixture and the resultant mixture was stirred for a whole day and night under reflux.
The solvent was distilled off from the reaction mixture thus obtained under reduced pressure. To the residue thus formed was added 300 ml of water and after washing the mixture with methylene chloride, the pH of the aqueous solution was adjusted to 4 with concentrated hydrochloric acid to deposit crystals, which were collected by filtration and recrystallized from ethanol to provide 36.1 g of the desired product.

Melting point: 158.5 to 159.5°C

Mass spectrum (m/z): 224 ($M^+$)

(It was confirmed by HPLC that trans/cis was 98.8/1.2).

Reference Example 26

Synthesis of ethyl 2-(2-(Z)-butenyl)-3-oxobutanoate

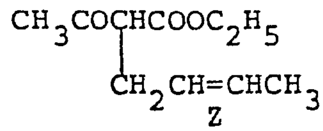

By following the same procedure as Reference Example 24 except that 1-chloro-2(Z)-butene was used in place of 1-chloro-2(E)-butene, the desired product was obtained.

Boiling point: 100 to 106°C/12 mm Hg

Mass spectrum (m/z): 185 ($M^+$ + 1)

(It was confirmed by $^{13}C$ NMR that trans/cis was 14.4/85.6)

Reference Example 27

<u>Synthesis of 5-(2-(Z)-butenyl)-6-methyl-2-nitroamino-4(1H)-pyrimidone</u>

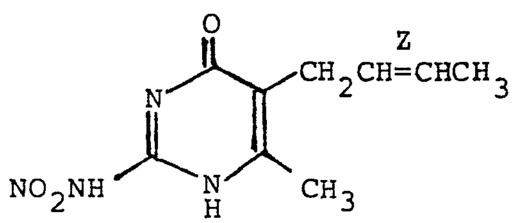

By following the same procedure as Reference Example 25 except that ethyl 2-(2-(Z)-butenyl)-3-oxobutanoate was used in place of 2-(2-(E)butenyl)-3-oxobutanoate, the desired product was obtained.

Melting point: 149.5 to 153.5°C

Mass spectrum (m/z): 224 ($M^+$)

(It was confirmed by HPLC that trans/cis was 2.7/97.3)

Reference Example 28

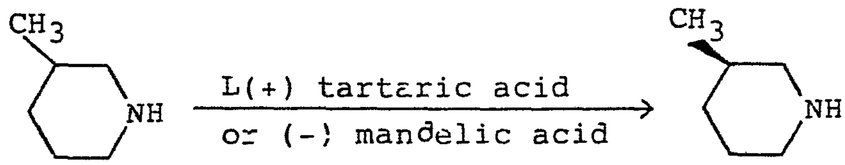


By optically resolving 3-methylpiperidine using L(+) tartaric acid or (-) mandelic acid based on the method shown in "Gazz. Chim. Ita., 102, 201(1972)" or "Naunyn-Schmiedeberg's Arch. of Pharmacol.", R-(-)-3-methylpiperidine was obtained.

Reference Example 29

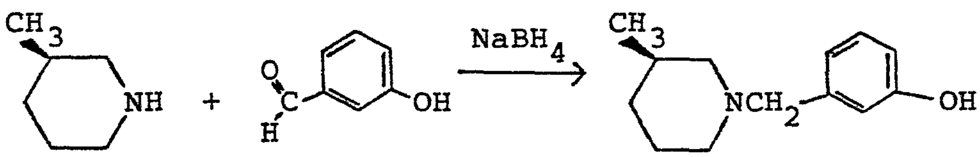


In 150 ml of ethanol were dissolved 10.66 g of R-(-)- 3-methylpiperidine and 10.93 g of 3-hydroxybenzaldehyde and then 3.38 g of sodium borohydride was added to the solution under ice-cooling. Then, the temperature of the mixture was allowed to rise to room temperature and the mixture was stirred for 20 hours. The solvent was distilled off under reduced pressure, the residue was dissolved in dilute hydrochloric acid, and after washing the solution thus obtained with ethyl acetate, the aqueous layer formed was recovered and alkalified by the addition of aqueous ammonia to deposit crystals, which were collected by filtration, washed with water, and then recrystallized from acetonitrile to provide 12.0 g of 3-[(R-(-)-3-methylpiperidino)-methyl]phenol having a melting point of 122 to 123°C and showing $[\alpha]_D^{23}$ - 14.3° (C = 2.3, $CHCl_3$).

Reference Example 30

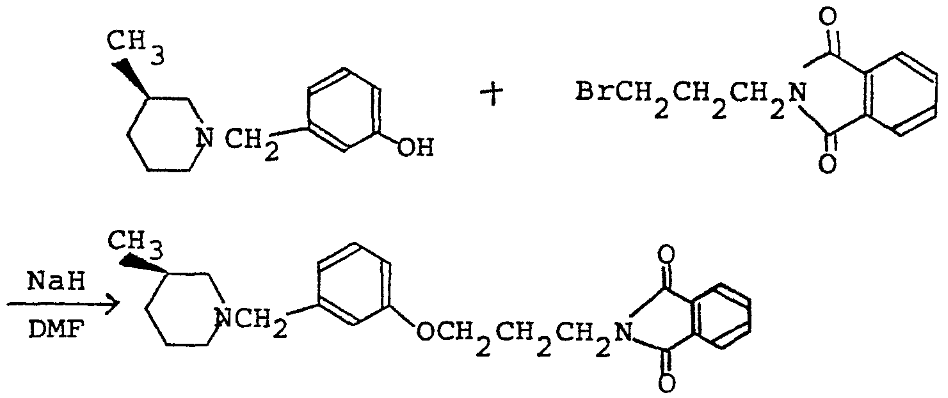


In 80 ml of desiccated dimethylformamide was suspended 2.5 g of 60% oily sodium hydride and after adding thereto 11.8 g of 3-[(R-(-)-3-methylpiperidino)methyl]-phenol under ice-cooling, the resultant mixture was stirred for 30 minutes at room temperature. Furthermore, 15.4 g of N-(3-bromopropyl)phthalimide was added to the mixture and after stirring the resultant mixture for 20 hours at 70°C, the solvent was distilled off under reduced pressure. The residue thus formed was dissolved in 300 ml of ethyl acetate and the solution thus formed was washed several times with water. The organic layer formed was recovered, dried with anhydrous magnesium sulfate, the solvent was distilled off under reduced pressure, and the residue thus formed was purified by silica gel column chromatography using a mixed solvent of ethyl acetate and n-hexane to provide 20.65 g of oily N-[3-[[3-(R-(-)-3-methylpiperidino)methyl]-phenoxy]propyl]phthalimide showing $[\alpha]_D^{23}$ - 5.90° (C =

1.9, $CHCl_3$).

Reference Example 31

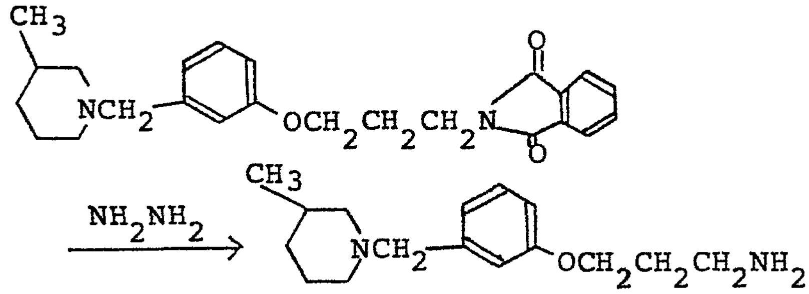


In 150 ml of ethanol was dissolved 20.65 g of N-[3-[[3-(R-(-)-3-methylpiperidino)methyl]phenoxy]propyl]-phthalimide and after adding thereto 2.6 g of hydrazine hydrate, the mixture was refluxed for 10 hours. Crystalline insoluble matters were removed from the reaction mixture by filtration, the mother liquid was concentrated, and the residue thus formed was purified by silica gel column chromatography using a mixed solvent of chloroform, methanol, and aqueous ammonia as a developing solvent to provide 3.24 g of oily 3-[3-(R-(-)-methylpiperidino)phenoxy]propylamine showing $[\alpha]_D^{23}$ - 8.83° (C = 7.5, $CHCl_3$).

Reference Example 32

Optical resolution of 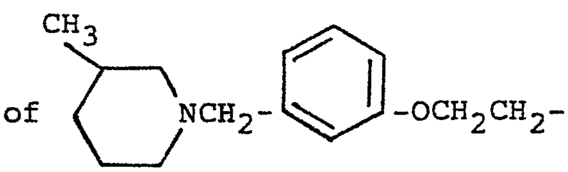 $CH_2NH_2$:

A salt product was formed from 16.5 g of 3-[3-(3-methylpiperidinomethyl)phenoxy]propylamine and 18.9 g of D(-) tartaric acid in methanol and the product was recrystallized 8 times from methanol to provide 4.02 g of 3-[3-(R-(-)-3-methylpiperidinomethyl)phenoxy]propylamine D(-) tartarate having a melting point of 159 to 160°C and showing

$[\alpha]_D^{23}$ - 20.58° (C = 1.02, $H_2O$).

The D(-) tartarate thus obtained was dissolved in water and after alkalifying the solution with an aqueous 10% sodium hydroxide solution, the product was extracted with ethyl acetate. The extract thus formed was dried by anhydrous potassium carbonate and then the solvent was distilled off under reduced pressure to provide 1.92 g of 3-[3-(R-(-)-3-methylpiperidinomethyl)-phenoxy]propylamine showing $[\alpha]_D^{23}$ - 8.87 (C = 7.92, $CHCl_3$).

Then, the first three mother liquids from which the crystals of 3-[3-(3-methylpiperidinomethyl)phenoxy]-propylamine D(-) tartarate were recovered were combined with each other and after distilling off the solvent therefrom under reduced pressure, the residue thus formed was dissolved in water. The aqueous solution thus obtained was alkalified with an aqueous 10% sodium hydroxide solution, the product was extracted with ethyl acetate, and after drying the extract with anhydrous potassium carbonate, the solvent was distilled off under reduced pressure to provide 8.89 g of 3-[3-(3-methylpiperidinomethyl)phenoxy]propylamine showing $[\alpha]_D^{23}$ + 3.2° (C = 2.68, $CHCl_3$). To the amine was added 10.17 g of L(+) tartaric acid in methanol to form a salt and the salt thus formed was recrystallized 6 times from methanol to provide 2.91 g of

3-[3-(S-(+)-3-methylpiperidinomethyl)phenoxy]propylamine L(+) tartarate of m.p. 158 to 160°C and showing

$[\alpha]_D^{23}$ - 21.43° (C = 1.96, $H_2O$).

From the tartarate was obtained 1.56 g of 3-[3-(S-(+)-3-methylpiperidinomethyl)phenoxy]propylamine showing $[\alpha]_D^{23}$ + 8.60° (C = 3.49, $CHCl_3$) in conventional manner.

Reference Example 33

(1)

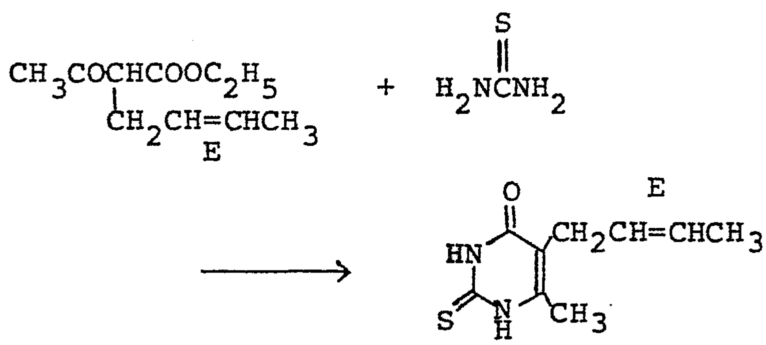

To 100 ml of methanol were added 42 ml of a methanol solution of 28% sodium methoxide and 8.26 g of thiourea; after refluxing the mixture for one hour, 20 g of ethyl 2-(2-(E)-butenyl)-3-oxobutanoate was added to the mixture at room temperature, and the resultant mixture was refluxed for 15 hours. The solvent was distilled off under reduced pressure and the residue was dissolved in 150 ml of water. The solution thus obtained was washed with methylene chloride and the pH thereof was adjusted to 6 with concentrated hydrochloric acid to deposit crystals, which were collected by filtration, washed with water, and dried to provide 14.43 g of 5-(2-(E)-butenyl-6-methyl-2-thiouracil.

The product showed a melting point of 226 to 227°C when recrystallized from ethanol.

(2)

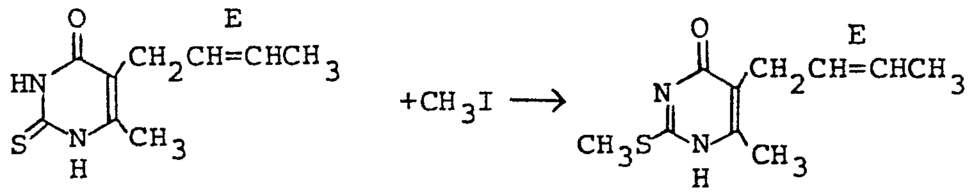


In 20 ml of water was dissolved 1.04 g of sodium hydroxide; 30 ml of ethanol and 5.0 g of 5-(2-(E)-butenyl)-6-methyl-2-thiouracil were added and dissolved, and after adding thereto 3.9 g of methyl iodide, the resultant mixture was heated to 60°C for 30 minutes.

Then, 20 ml of water was added to the reaction mixture and the mixture was ice-cooled to deposit crystals, which were collected by filtration and washed with water to provide 4.51 g of 5-(2-(E)-butenyl)-6-methyl-2-methylthio-4(1H)-pyrimidone of melting point 191 to 192°C.

Reference Example 34

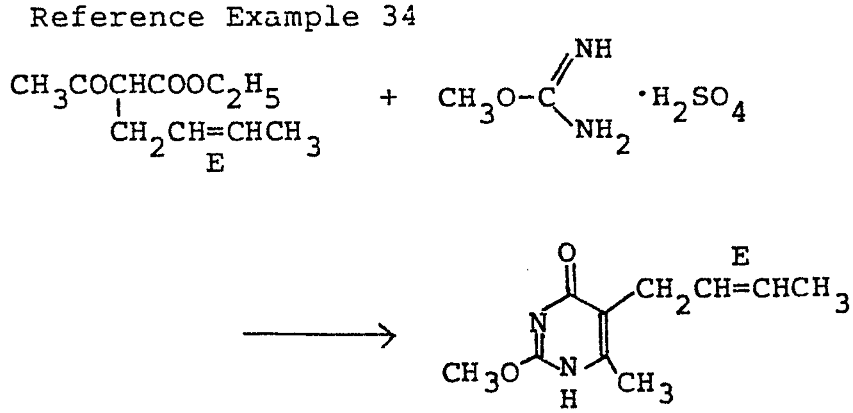

In 150 ml of methanol were dissolved 11.66 g of 2-(2-(E)-butenyl)-3-oxobutanoate and 10.9 g of o-methylisourea sulfate and 36.6 ml of a methanol solution of

28% sodium methylate was added dropwise to the solution under ice-cooling. Thereafter, the temperature of the mixture was allowed to rise to room temperature and after stirring the mixture for 24 hours at room temperature, the solvent was distilled off under reduced pressure. The residue thus formed was added to 100 ml of ice-water and the pH of the mixture was adjusted to 6 to 7 with dilute hydrochloric acid to deposit crystals, which were collected by filtration and recrystallized from methanol to provide 3.0 g of 5-(2-(E)-butenyl)-6-methyl-2-methoxy-4(1H)-pyrimidone of melting point 200 to 201°C.

Reference Example 35

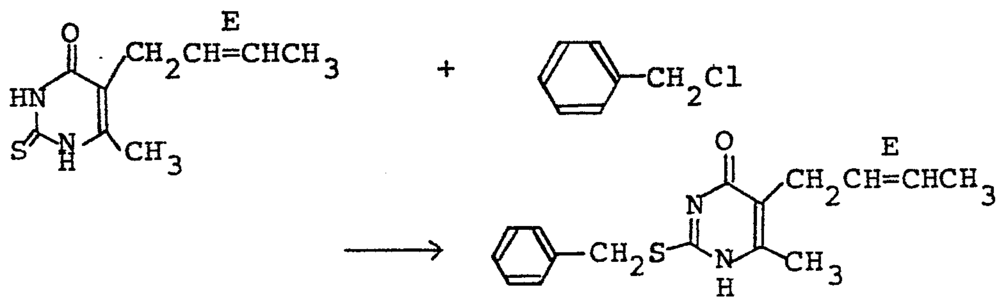

By following the same procedure as Reference Example 33(2) except that benzyl chloride was used in place of methyl iodide, 5-(2-(E)-butenyl)-6-methyl-2-benzylthio-4(1H)-pyrimidone of melting point 154 to 155°C was obtained.

Example 1

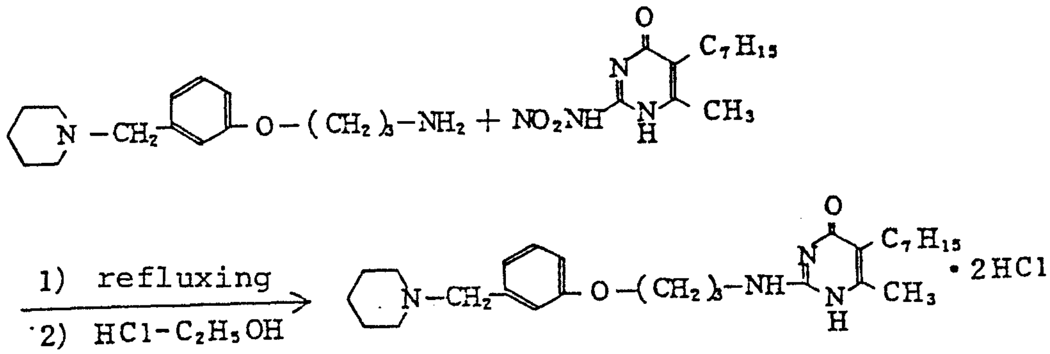


To 40 ml of pyridine were added 1.49 g of 3-[m-(piperidinomethyl)phenoxy]propylamine and 1.48 g of 5-heptyl-6-methyl-2-nitroamino-4-(1H)-pyrimidone and the mixture was stirred for 60 hours under reflux. The solvent was distilled off from the reaction mixture under reduced pressure and the residue was purified by column chromatography (silica gel, eluent: chloroform-methanol). The product thus obtained was treated by ethanolic hydrogen chloride to form crystals, which were recrystallized from acetonitrile to provide 2.25 g of 5-heptyl-6-methyl-2-[3-[m-(piperidinomethyl)phenoxy]-propylamino]-4(1H)-pyrimidone di-hydrochloride showing a melting point of 170.0 to 174.0°C.

Elemental analysis for $C_{27}H_{42}N_4O_2.2HCl$

| | C | H | N | Cl |
|---|---|---|---|---|
| Caculated: | 61.47% | 8.41% | 10.62% | 13.44% |
| Found: | 61.24% | 8.63% | 10.79% | 13.52% |

Mass spectrum (EI)

m/z: 454 ($M^+$)

By following the procedure of Example 1,

the following compounds were obtained (Examples 2 and 3):

Example 2

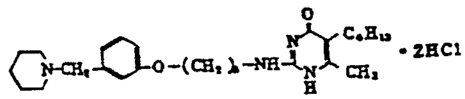

5-Hexyl-6-methyl-2-[3-[m-(piperidinomethyl)phenoxy]-propylamino]-4-(1H)-pyrimidone dihydrochloride

Physicochemical properties :

(i) Melting point 162.0-165.0°C

(ii) Elemental analysis for $C_{26}H_{40}N_4O_2.2HCl$:

| | C | H | N | Cl |
|---|---|---|---|---|
| Calculated: | 60.81% | 8.24% | 10.91% | 13.81% |
| Found: | 60.41% | 8.32% | 10.94% | 13.80% |

(iii) Mass spectrum (EI)

m/z: 440 ($M^+$)

Example 3

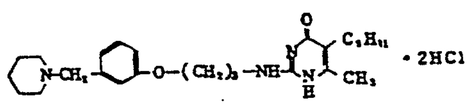

6-Methyl-5-pentyl-α-[3-[m-(piperidinomethyl)-phenoxy]propylamino]-4-(1H)-pyrimidone dihydrochloride:

Physicochemical properties:

(i) Melting point 170.0-175.0°C

(ii) Elemental analysis for $C_{25}H_{38}N_4O_2.2HCl$:

| | C | H | N | Cl |
|---|---|---|---|---|
| Calculated: | 60.11% | 8.07% | 11.22% | 14.19% |
| Found: | 59.85% | 8.25% | 11.20% | 14.34% |

(iii) Mass spectrum (EI)

m/z: 426 ($M^+$)

Example 4

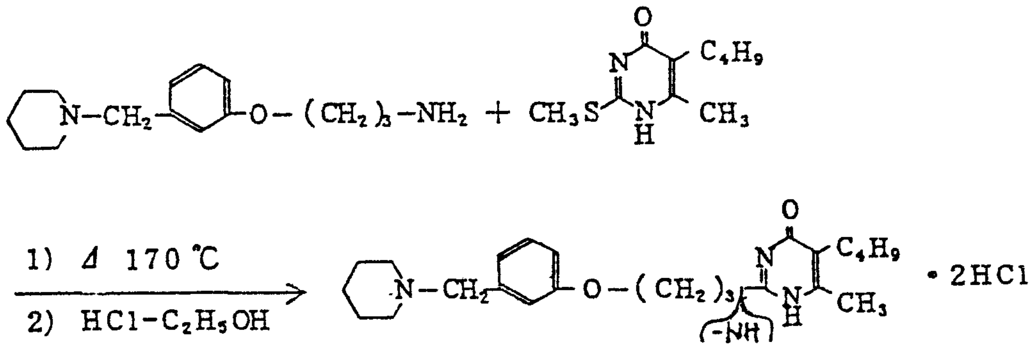


After reacting 1.8 g of 3-[m-(piperidinomethyl)-phenoxy] propylamine and 1.5 g of 5-butyl-6-methyl-2-methylthio-4(1H)-pyrimidone for 5 hours at 170°C, the reaction product thus obtained was purified by column chromatography (silica gel, eluent: chloroform-methanol). The product was treated with ethanolic HCl to form crystals, which were recrystallized from ethanol-ethyl acetate to provide 1.0 g of 5-butyl-6-methyl-2-[3-[m-(piperidinomethyl)phenoxy]propylamino]-4(1H)-pyrimidone dihydrochloride showing a melting point of 171.0 to 174.0°C.

Elemental analysis for $C_{24}H_{36}N_4O_2.2HCl$:

| | C | H | N | Cl |
|---|---|---|---|---|
| Calculated: | 59.38% | 7.89% | 11.54% | 14.60% |
| Found: | 59.11% | 8.17% | 11.42% | 14.58% |

Mass spectrum (EI)

m/z: 412 ($M^+$)

By following the procedure of Example 1, the following compounds were obtained (Examples 5 to 7):

Example 5

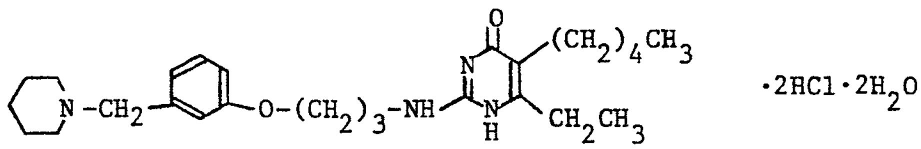

6-Ethyl-5-pentyl-2-[3-[m-(piperidinomethyl)phenoxy]-propylamino]-4(1H)-pyrimidone dihydrochloride· dihydrate

(i) m.p. 106 - 108°C (recrystallized from ether-hexane)

(ii) Elemental analysis for $C_{26}H_{40}N_4O_2 \cdot 2HCl \cdot 2H_2O$

| | C(%) | H(%) | N(%) | Cl(%) |
|---|---|---|---|---|
| Calculated: | 56.82 | 8.44 | 10.19 | 12.90 |
| Found: | 56.70 | 8.48 | 10.17 | 13.03 |

(iii) Mass spectrum (FAB): (m/z) 441 ($M^+$+1)

Example 6

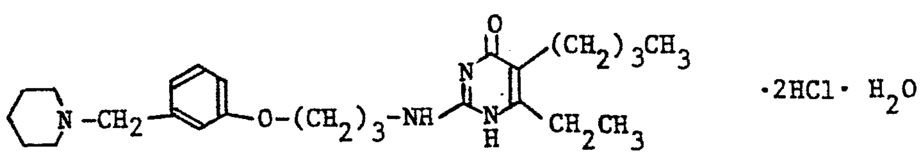

5-Butyl-6-ethyl-2-[3-[m-(piperidinomethyl)phenoxy]-propylamino]-4(1H)-pyrimidone dihydrochloride monohydrate

(i) m.p. 110 - 122°C (recrystallized from ether-acetonitrile)

(ii) Elemental analysis for $C_{25}H_{38}N_4O_2 \cdot 2HCl \cdot H_2O$

| | C(%) | H(%) | N(%) |
|---|---|---|---|
| Calculated: | 58.02 | 8.18 | 10.83 |
| Found: | 57.78 | 8.11 | 10.80 |

(iii) Mass spectrum (EI): (m/z) 426 ($M^+$)

Example 7

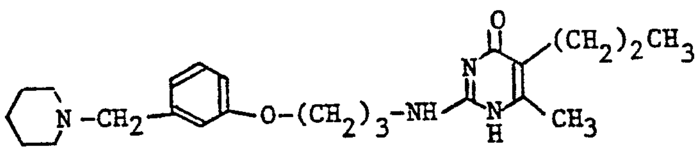

6-Methyl-2-[3-[m-(piperidinomethyl)phenoxy]propylamino]-5-propyl-4(1H)-pyrimidone

(i) m.p. 96 - 97°C (recrystallized from ethanol-acetonitrile)

(ii) Elemental analysis for $C_{23}H_{34}N_4O_2$

| | C(%) | H(%) | N(%) |
|---|---|---|---|
| Calculated: | 69.32 | 8.60 | 14.06 |
| Found: | 69.09 | 8.85 | 14.12 |

(iii) Mass spectrum (EI): (m/z) 398 ($M^+$)

Example 8

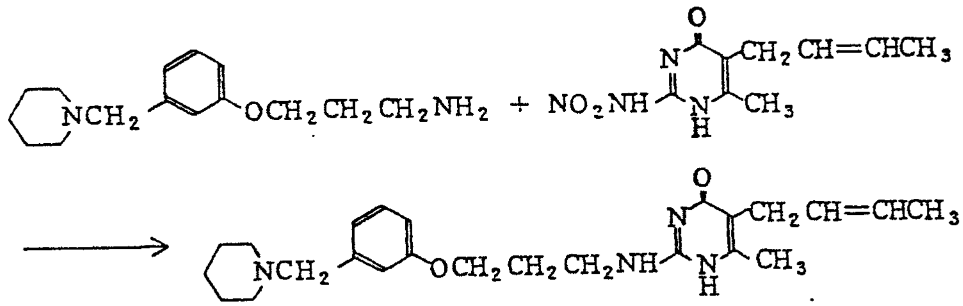

After refluxing 1.66 g of 3-[m-(piperidinomethyl)-phenoxy]propylamine and 1.5 g of 2-nitroamino-5-(2-butenyl)-6-methyl-4(1H)-pyrimidone in 40 ml of pyridine for 48 hours, the solvent was distilled off under reduced pressure. The residue thus formed was purified by silica gel column chromatography using a mixed solvent of chloroform and methanol as a developing solvent and the product was recrystallized from a mixture of ethanol and acetonitrile to provide 1.05 g of 5-(2-butenyl)-6-methyl-2-[3-[m-(piperidinomethyl)phenoxy]-propylamino]-4(1H)-pyrimidone showing a melting point of 110 to 111°C.

Elemental analysis for $C_{24}H_{34}N_4O_2 \cdot 1/2H_2O$

| | C | H | N |
|---|---|---|---|
| Calculated: | 68.71% | 8.41% | 13.35% |
| Found: | 68.88% | 8.43% | 13.41% |

Mass spectra:

(m/z): 410 ($M^+$), 355, 327

By following the same procedure as above, the following compounds were obtained (Examples 9 to 38) :

Example 9

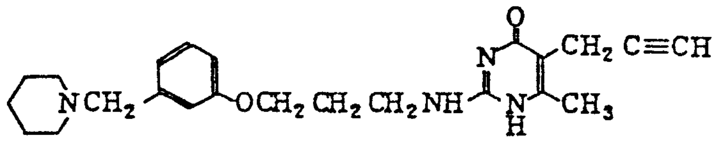

6-Methyl-2-[3-[m-(piperidinomethyl)phenoxy]propylamino]-5-(2-propynyl)-4(1H)-pyrimidone

(i) m.p. 179 - 180°C

(ii) Elemental analysis for $C_{23}H_{30}N_4O_2 \cdot 1/2H_2O$

| | C(%) | H(%) | N(%) |
|---|---|---|---|
| Calculated: | 68.46 | 7.74 | 13.88 |
| Found: | 68.77 | 7.88 | 13.94 |

(iii) Mass spectra: (m/z) 394 ($M^+$), 311

Example 10

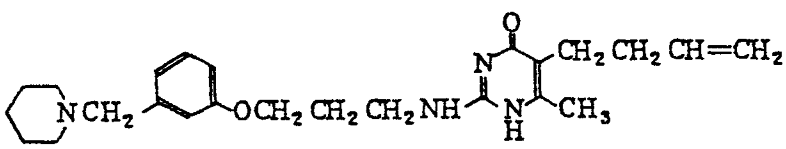

5-(3-Butenyl)-6-methyl-2-[3-[m-(piperidinomethyl)-phenoxy]propylamino]-4(1H)-pyrimidone

(i) m.p. 84 - 85°C

(ii) Elemental analysis for $C_{24}H_{34}N_4O_2$

| | C(%) | H(%) | N(%) |
|---|---|---|---|
| Calculated: | 70.21 | 8.35 | 13.65 |
| Found: | 70.41 | 8.49 | 13.64 |

(iii) Mass spectra: (m/z) 410 ($M^+$), 369, 327

Example 11

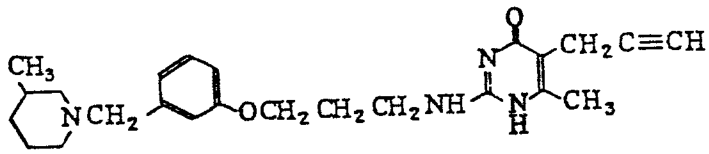

6-Methyl-2— [3-[m-(3-methylpiperidinomethyl)phenoxy]-propylamino]-5-(2-propynyl)-4(1H)-pyrimidone

(i) m.p. 149.5 - 150°C

(ii) Elemental analysis for $C_{24}H_{32}N_4O_2$

| | C(%) | H(%) | N(%) |
|---|---|---|---|
| Calculated: | 70.56 | 7.89 | 13.71 |
| Found: | 70.64 | 7.88 | 13.77 |

(iii) Mass spectra: (m/z) 408 ($M^+$), 365, 311

Example 12

(a) 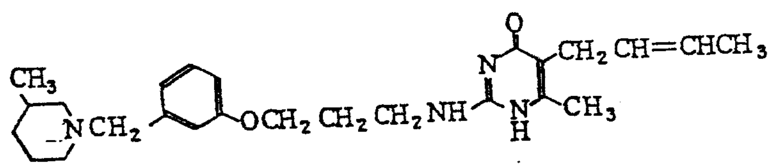

5-(2-Butenyl)-6-methyl-2-[3-[m-(3-methylpiperidino-methyl)phenoxy]propylamino]-4(1H)-pyrimidone

(i) m.p. 98 - 99°C

(ii) Elemental analysis for $C_{25}H_{36}N_4O_2$

| | C(%) | H(%) | N(%) |
|---|---|---|---|
| Calculated: | 70.72 | 8.55 | 13.20 |
| Found: | 70.74 | 8.73 | 13.19 |

(iii) Mass spectra: (m/z) 424 ($M^+$), 381, 327

(b) 5-(2-Butenyl)-6-methyl-2-[3-[m-(3-methylpiperidinomethyl)phenoxy]propylamino]-4(1H)-pyrimidone dihydrochloride

(i) m.p. 159 - 161°C

(ii) Elemental analysis for $C_{25}H_{38}N_4O_2Cl_2$

| | C(%) | H(%) | N(%) | Cl(%) |
|---|---|---|---|---|
| Calculated: | 60.36 | 7.70 | 11.26 | 14.25 |
| Found: | 60.16 | 7.96 | 11.19 | 14.25 |

(iii) Mass spectra: (m/z) 424($M^+$), 369, 327

(c) 5-(2-Butenyl)-6-methyl-2-[3-[m-(3-methylpiperidinomethyl)phenoxy]propylamino]-4(1H)-pyrimidone maleate

(i) m.p. 120 - 122°C

(ii) Elemental analysis for $C_{29}H_{40}N_4O_6$

| | C(%) | H(%) | N(%) |
|---|---|---|---|
| Calculated: | 64.42 | 7.46 | 10.36 |
| Found: | 64.48 | 7.40 | 10.33 |

(iii) Mass spectra: (m/z) 424($M^+$), 369, 327

Example 13

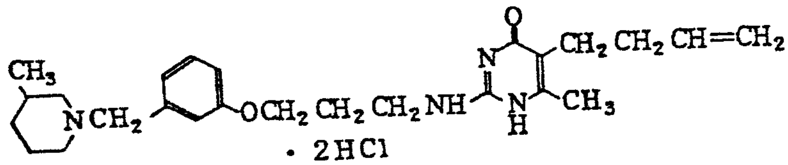

· 2HCl

5-(3- Butenyl)-6-methyl-2-[3-[m-(3-methylpiperidinomethyl)phenoxy]propylamino]-4(1H)-pyrimidone dihydrochloride

(i) m.p. 147 - 150°C

(ii) Elemental analysis for $C_{25}H_{38}N_4O_2Cl_2$

| | C(%) | H(%) | N(%) | Cl(%) |
|---|---|---|---|---|
| Calculated: | 60.36 | 7.70 | 11.26 | 14.25 |
| Found: | 60.11 | 7.47 | 11.50 | 14.28 |

(iii) Mass spectra: (m/z) 424 ($M^+$), 383, 327

Example 14

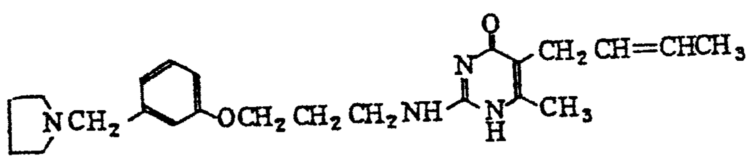

5-(2-Butenyl)-6-methyl-2-[3-[m-(pyrrolidinylmethyl)-phenoxy]propylamino]-4(1H)-pyrimidone

(i) m.p. 132 - 133°C

(ii) Elemental analysis for $C_{23}H_{32}N_4O_2$

| | C(%) | H(%) | N(%) |
|---|---|---|---|
| Calculated: | 69.67 | 8.13 | 14.13 |
| Found: | 69.90 | 8.40 | 14.09 |

(iii) Mass spectra: (m/z) 396 ($M^+$), 381, 340, 327

Example 15

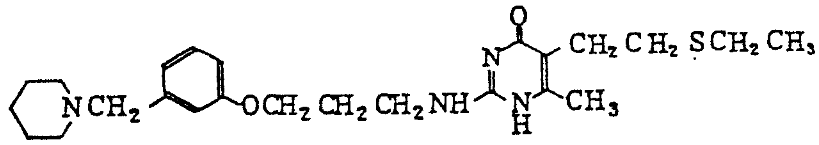

5-(2-Ethylthioethyl)-6-methyl-2-[3-[m-(piperidinomethyl)phenoxy]propylamino]-4(1H)-pyrimidone

(i) m.p. 94 - 95°C

(ii) Elemental analysis for $C_{24}H_{36}N_4O_2S$

| | C(%) | H(%) | N(%) | S(%) |
|---|---|---|---|---|
| Calculated: | 64.83 | 8.16 | 12.60 | 7.2 |
| Found: | 64.76 | 8.46 | 12.44 | 6.8 |

(iii) Mass spectra: (m/z) 444 ($M^+$), 415, 383, 369, 361

Example 16

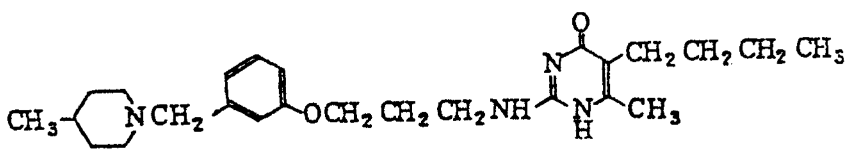

5-Butyl-6-methyl-2-[3-[m-(4-methylpiperidinomethyl)-phenoxy]propylamino]-4(1H)-pyrimidone

(i) m.p. 104.5 - 105 5°C

(ii) Elemental analysis for $C_{25}H_{38}N_4O_2$

| | C(%) | H(%) | N(%) |
|---|---|---|---|
| Calculated: | 70.39 | 8.98 | 13.13 |
| Found: | 70.35 | 9.12 | 13.24 |

(iii) Mass spectra: (m/z) 426 ($M^+$), 397, 369, 329

Example 17

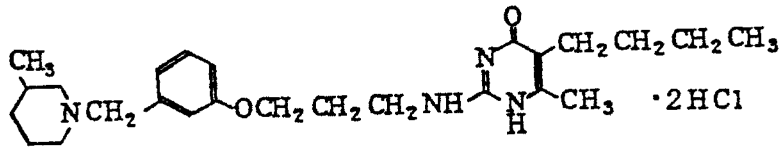

5-Butyl-6-methyl-2-[3-[m-(3-methylpiperidinomethyl)-phenoxy]propylamino]-4(1H)-pyrimidone dihydrochloride

(i) m.p. 155 - 158°C

(ii) Elemental analysis for $C_{25}H_{40}N_4O_2Cl_2$

| | C(%) | H(%) | N(%) | Cl(%) |
|---|---|---|---|---|
| Calculated: | 60.11 | 8.07 | 11.22 | 14.19 |
| Found: | 59.78 | 7.91 | 12.13 | 13.97 |

(iii) Mass spectra: (m/z) 426 ($M^+$), 329

Example 18

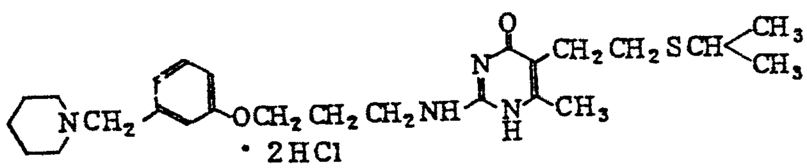

5-(2-Isopropylthioethyl)-6-methyl-2-[3-[m-(piperidinomethyl)phenoxy]propylamino]-4(1H)-pyrimidone dihydrochloride

(i) m.p. 159 - 162°C

(ii) Elemental analysis for $C_{25}H_{40}N_4O_2SCl_2$

| | C(%) | H(%) | N(%) | S(%) | Cl(%) |
|---|---|---|---|---|---|
| Calculated: | 56.49 | 7.58 | 10.54 | 6.03 | 13.34 |
| Found: | 56.31 | 7.79 | 10.57 | 5.83 | 13.14 |

(iii) Mass spectra: (m/z) 458 ($M^+$), 415

Example 19

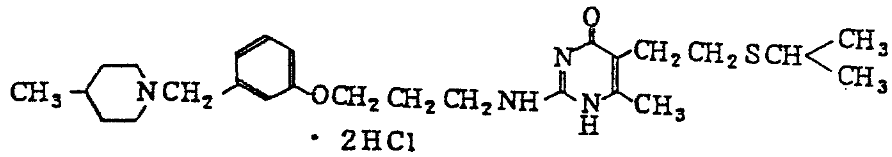

5-(2-Isopropylthioethyl)-6-methyl-2-[3-[m-(4-methylpiperidinomethyl)phenoxy]propylamino]-4(1H)-pyrimidone dihydrochloride

(i) m.p. 153 - 156°C

(ii) Elemental analysis for $C_{26}H_{42}N_4O_2SCl_2$

| | C(%) | H(%) | N(%) | S(%) | Cl(%) |
|---|---|---|---|---|---|
| Calculated: | 57.24 | 7.76 | 10.27 | 5.88 | 13.00 |
| Found: | 57.06 | 7.72 | 10.21 | 5.63 | 13.01 |

(iii) Mass spectra: (m/z) 472($M^+$), 429

Example 20

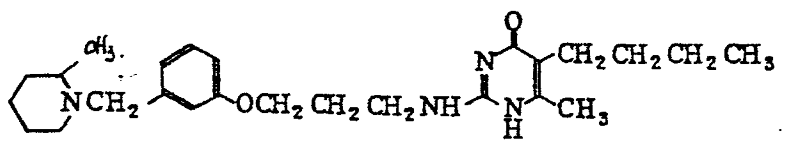

5-Butyl-6-methyl-2-[3-[m-(2-methylpiperidinomethyl)phenoxy]propylamino]-4(1H)-pyrimidone

(i) m.p. 89 - 90°C

(ii) Elemental analysis for $C_{25}H_{38}N_4O_2$

| | C(%) | H(%) | N(%) |
|---|---|---|---|
| Calculated: | 70.39 | 8.98 | 13.13 |
| Found: | 70.30 | 9.13 | 13.22 |

(iii) Mass spectra: (m/z) 426($M^+$), 411, 329

Example 21

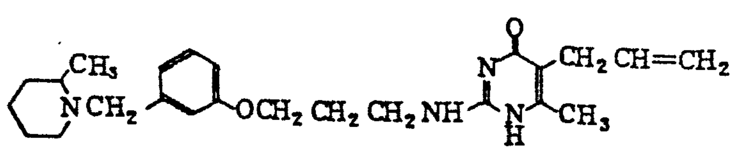

5-(2-propenyl)-6-methyl-2-[3-[m-(2-methylpiperidinomethyl)phenoxy]propylamino]-4(1H)-pyrimidone

(i) m.p. 124 - 125°C

(ii) Elemental analysis for $C_{24}H_{34}N_4O_2$

| | C(%) | H(%) | N(%) |
|---|---|---|---|
| Calculated: | 70.21 | 8.35 | 13.65 |
| Found: | 70.42 | 8.55 | 13.68 |

(iii) Mass spectra: (m/z) 410($M^+$), 395, 313

Example 22

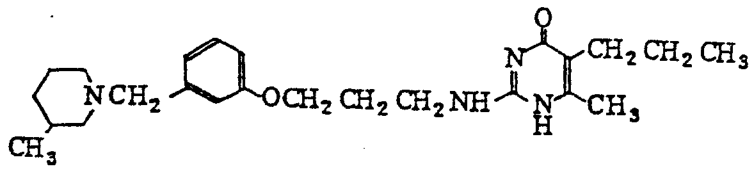

6-methyl-2-[3-[m-(3-methylpiperidinomethyl)phenoxy]propylamino]-5-propyl-4(1H)-pyrimidone

(i) m.p. 129 - 130°C (recrystallized from ethanol - acetonitrile)

(ii) Elemental analysis for $C_{24}H_{36}N_4O_2$

| | C(%) | H(%) | N(%) |
|---|---|---|---|
| Calculated: | 69.87 | 8.79 | 13.58 |
| Found: | 69.99 | 8.84 | 13.68 |

(iii) Mass spectrum(EI): (m/z) 412($M^+$)

Example 23

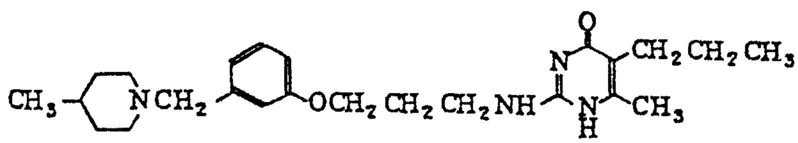

6-methyl-2-[3-[m-(4-methylpiperidinomethyl)phenoxy]propylamino]-5-propyl-4(1H)-pyrimidone

(i) m.p. 89 - 90°C (recrystallized from ethanol - acetonitrile)

(ii) Elemental analysis for $C_{24}H_{36}N_4O_2$

| | C(%) | H(%) | N(%) |
|---|---|---|---|
| Calculated: | 69.87 | 8.79 | 13.58 |
| Found: | 69.60 | 8.85 | 13.58 |

(iii) Mass spectrum(EI): (m/z) 412($M^+$)

Example 24

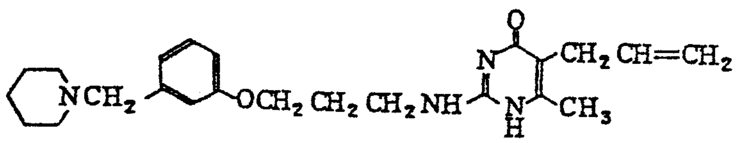

5-(2-propenyl)-6-methyl-2-[3-[m-(piperidinomethyl)phenoxy]propylamino]-4(1H)-pyrimidone

(i) m.p. 138 - 140°C(recrystallized from ethanol - acetonitrile)

(ii) Elemental analysis for $C_{23}H_{32}N_4O_2$

| | C(%) | H(%) | N(%) |
|---|---|---|---|
| Calculated: | 69.67 | 8.13 | 14.13 |
| Found: | 69.49 | 8.30 | 14.22 |

(iii) Mass spectrum(EI): (m/z) 396($M^+$)

Example 25

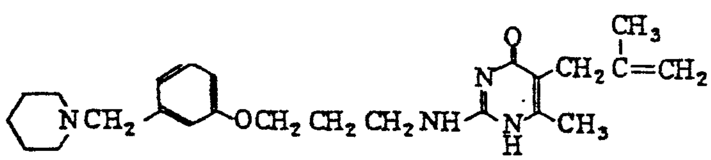

6-methyl-5-(2-methyl-2-propenyl)-2-[3-[m-(piperidinomethyl)phenoxy]propylamino]-4(1H)-pyrimidone

(i) m.p. 127 - 129°C(recrystallized from ethanol - acetonitrile)

(ii) Elemental analysis for $C_{24}H_{34}N_4O_2$

| | C(%) | H(%) | N(%) |
|---|---|---|---|
| Calculated: | 70.21 | 8.35 | 13.65 |
| Found: | 70.24 | 8.53 | 13.64 |

(iii) Mass spectrum(EI): (m/z) 410($M^+$)

Example 26

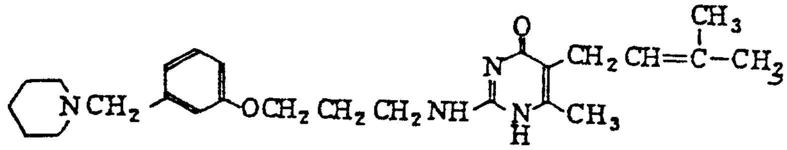

6-methyl-5-(3-methyl-2-butenyl)-2-[3-[m-(piperidinomethyl)phenoxy]propylamino]-4(1H)-pyrimidone

(i) m.p. 132 - 134°C(recrystallized from ethanol - acetonitrile)

(ii) Elemental analysis for $C_{25}H_{36}N_4O_2$

| | C(%) | H(%) | N(%) |
|---|---|---|---|
| Calculated: | 70.72 | 8.55 | 13.20 |
| Found: | 70.61 | 8.46 | 13.51 |

(iii) Mass spectrum(EI): (m/z) 424($M^+$)

Example 27

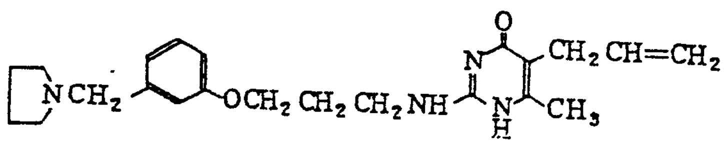

5-(2-propenyl)-6-methyl-2-[3-[m-(pyrrolidinylmethyl)phenoxy]propylamino]-4(1H)-pyrimidone

(i) m.p. 105 - 107°C(recrystallized from ethanol - acetonitrile)

(ii) Elemental analysis for $C_{22}H_{30}N_4O_2$

| | C(%) | H(%) | N(%) |
|---|---|---|---|
| Calculated: | 69.08 | 7.91 | 14.65 |
| Found: | 69.20 | 8.15 | 14.69 |

(iii) Mass spectrum(EI): (m/z) 382($M^+$)

Example 28

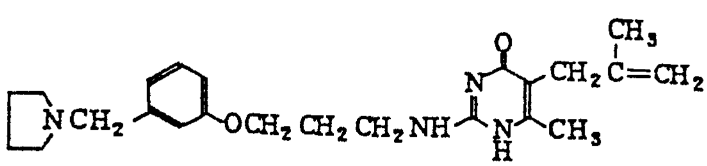

6-Methyl-5-(2-methyl-2-propenyl)-2-[3-[m-(pyrrolidinylmethyl)phenoxy]propylamino]-4(1H)-pyrimidone

(i) m.p. 112 - 114°C(recrystallized from ethanol - acetonitrile)

(ii) Elemental analysis for $C_{23}H_{32}N_4O_2$

| | C(%) | H(%) | N(%) |
|---|---|---|---|
| Calculated: | 69.67 | 8.13 | 14.13 |
| Found: | 69.58 | 8.38 | 14.28 |

(iii) Mass spectrum(EI): (m/z) 396($M^+$)

Example 29

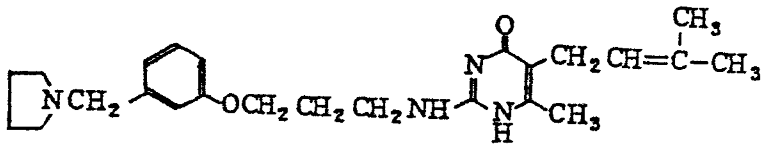

6-Methyl-5-(3-methyl-2-butenyl)-2-[3-[m-(pyrrolidinylmethyl)phenoxy]propylamino]-4(1H)-pyrimidone

(i) m.p. 114 - 116°C(recrystallized from ethanol - acetonitrile)

(ii) Elemental analysis for $C_{24}H_{34}N_4O_2$

| | C(%) | H(%) | N(%) |
|---|---|---|---|
| Calculated: | 70.21 | 8.35 | 13.65 |
| Found: | 70.21 | 8.44 | 13.73 |

(iii) Mass spectrum(EI): (m/z) 410($M^+$)

Example 30

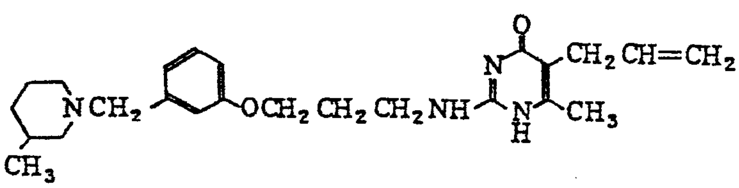

5-(2-Propenyl)-6-methyl-2-[3-[m-(3-methylpiperidinomethyl)phenoxy]propylamino]-4(1H)-pyrimidone

(i) m.p. 110 - 112°C(recrystallized from ethanol - acetonitrile)

(ii) Elemental analysis for $C_{24}H_{34}N_4O_2$

| | C(%) | H(%) | N(%) |
|---|---|---|---|
| Calculated: | 70.21 | 8.35 | 13.65 |
| Found: | 70.37 | 8.37 | 13.75 |

(iii) Mass spectrum(EI): (m/z) 410($M^+$)

Example 31

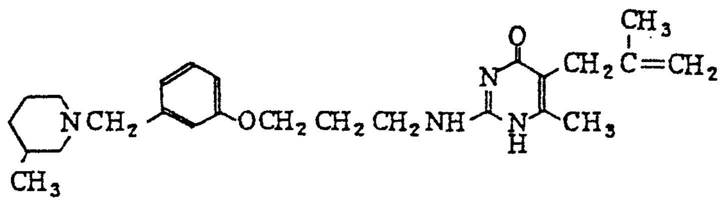

6-Methyl-2-[3-[m-(3-methylpiperidinomethyl)phenoxy]propylamino]-5-(2-methyl-2-propenyl)-4(1H)-pyrimidone

(i) m.p. 112 - 114°C(recrystallized from ethanol - acetonitrile)

(ii) Elemental analysis for $C_{25}H_{36}N_4O_2$

| | C(%) | H(%) | N(%) |
|---|---|---|---|
| Calculated: | 70.72 | 8.55 | 13.20 |
| Found: | 70.71 | 8.81 | 13.36 |

(iii) Mass spectrum(EI): (m/z) 424($M^+$)

Example 32

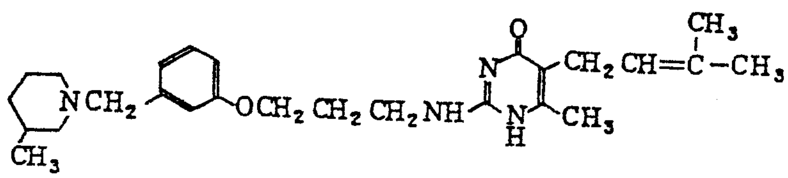

6-Methyl-5-(3-methyl-2-butenyl)-2-[3-[m-(3-methylpiperidinomethyl)phenoxy]propylamino]-4(1H)-pyrimidone

(i) m.p. 108 - 109°C(recrystallized from ethanol - acetonitrile)

(ii) Elemental analysis for $C_{26}H_{38}N_4O_2$

| | C(%) | H(%) | N(%) |
|---|---|---|---|
| Calculated: | 71.20 | 8.73 | 12.77 |
| Found: | 71.46 | 9.03 | 12.93 |

(iii) Mass spectrum(EI): (m/z) 438($M^+$)

Example 33

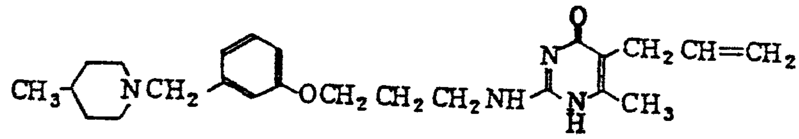

5-(2-Propenyl)-6-methyl-2-[3-[m-(4-methylpiperidinomethyl)phenoxy]propylamino]-4(1H)-pyrimidone

(i) m.p. 113 - 114°C(recrystallized from ethanol - acetonitrile)

(ii) Elemental analysis for $C_{24}H_{34}N_4O_2$

| | C(%) | H(%) | N(%) |
|---|---|---|---|
| Calculated: | 70.21 | 8.35 | 13.65 |
| Found: | 70.22 | 8.46 | 13.77 |

Mass spectrum(EI): (m/z) 410($M^+$)

Example 34

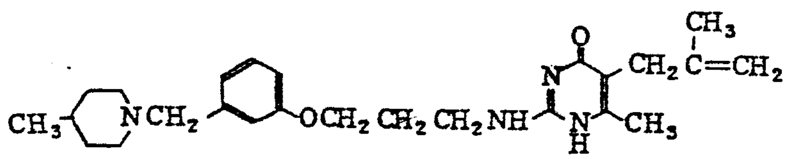

6-Methyl-2-[3-[m-(4-methylpiperidinomethyl)phenoxy]propylamino]-5-(2-methyl-2-propenyl)-4(1H)-pyrimidone

(i) m.p. 91 - 92°C(recrystallized from ethanol - acetonitrile)

(ii) Elemental analysis for $C_{25}H_{36}N_4O_2$

| | C(%) | H(%) | N(%) |
|---|---|---|---|
| Calculated: | 70.72 | 8.55 | 13.20 |
| Found: | 70.69 | 8.78 | 13.26 |

(iii) Mass spectrum(EI): (m/z) 424($M^+$)

Example 35

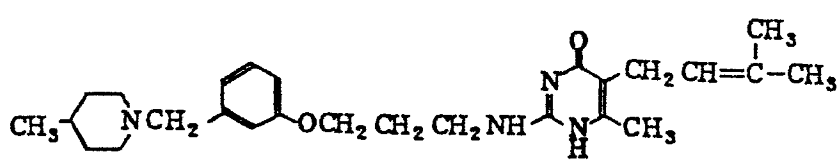

6-Methyl-5-(3-methyl-2-butenyl)-2-[3-[m-(4-methylpiperidinomethyl)phenoxy]propylamino]-4(1H)-pyrimidone

(i) m.p. 113 - 115°C (recrystallized from ethanol - acetonitrile)

(ii) Elemental analysis for $C_{25}H_{38}N_4O_2$

| | C(%) | H(%) | N(%) |
|---|---|---|---|
| Calculated: | 71.20 | 8.73 | 12.77 |
| Found: | 71.13 | 8.77 | 12.78 |

(iii) Mass spectrum(EI): (m/z) 438($M^+$)

Example 36

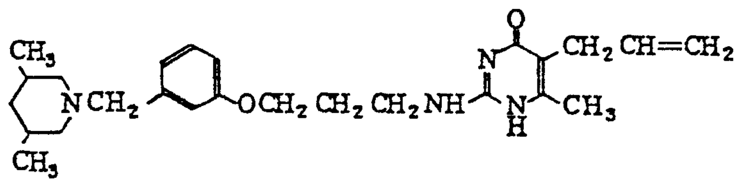

6-Methyl-2-[3-[m-(3,5-dimethylpiperidinomethyl)-phenoxy]propylamino]-5-(2-propenyl)-4(1H)-pyrimidone

(i) m.p. 111 - 114°C

(ii) Mass spectra: (m/z) 424($M^+$), 381

Example 37

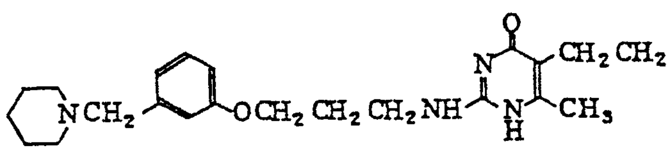

·2HCl

6-Methyl-2-[3-[m-(piperidinomethyl)phenoxy]propylamino]-5-(3,4,4-trifluoro-3-butenyl)-4(1H)-pyrimidone dihydrochoride

(i) m.p. 154 - 156°C

(ii) Mass spectra: (m/z) 464($M^+$), 381

Example 38

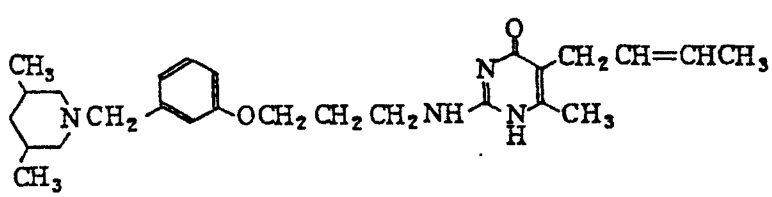

5-(2-Butenyl)-6-methyl-2-[3-[m-(3,5-dimethylpiperidinomethyl)phenoxy]propylamino]-4(1H)-pyrimidone

(i) m.p. 111 - 112°C

(ii) Mass spectra: (m/z) 438($M^+$), 395, 327

Example 39

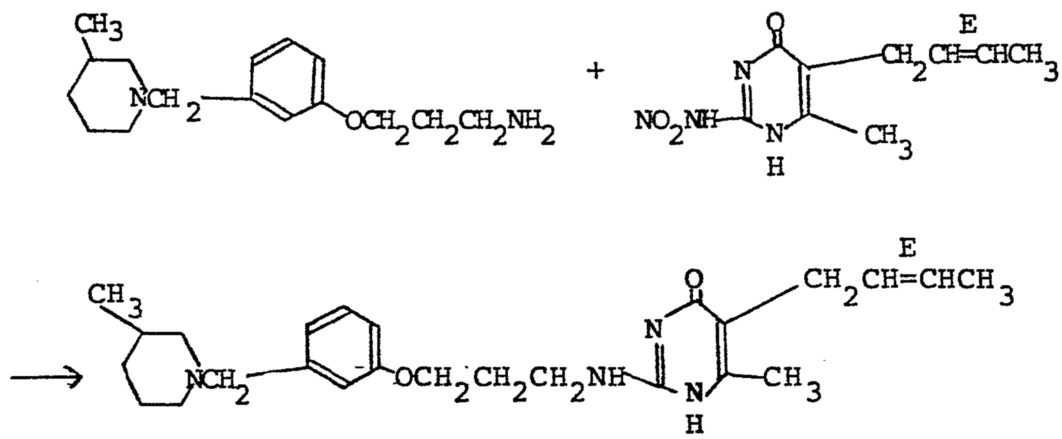

In 120 ml of toluene were refluxed 14.96 g of 3-[3-(3-methylpiperidinomethyl)phenoxy]propylamine and 12.06 g of 5-(2-(E)-butenyl)-6-methyl-2-nitroamino-4(1H)-pyrimidone for 20 hours. The solvent was distilled off under reduced pressure and the residue was dissolved in 110 ml of acetonitrile containing 20% ethanol and the solution was allowed to stand to deposit crystals, which were collected by filtration and recrystallized from 130 ml of acetonitrile containing 30% ethanol to provide 15.8 g of 5-(2-(E)-butenyl)-6-methyl-2- [3-[m-methylpiperidinomethyl)phenoxy]-propylamino]-4(1H)-pyrimidone of melting point 98 to 99.5°C.

Elemental analysis for $C_{25}H_{36}N_4O_2$:

| | C | H | N |
|---|---|---|---|
| Calculated: | 70.72% | 8.55% | 13.20% |
| Found: | 70.62% | 8.70% | 13.21% |

Example 40

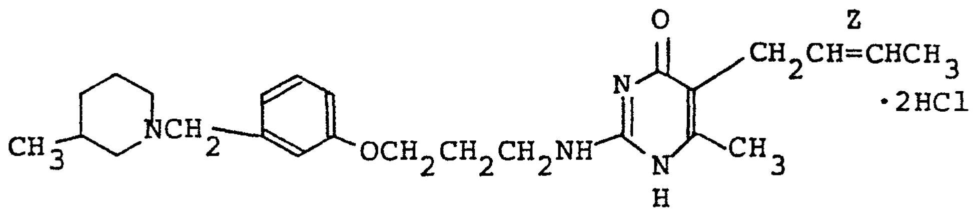

Synthesis of 5-(2-(Z)-butenyl)-6-methyl-2- [3-[m-[(3-methyl)piperidinomethyl]phenoxy]propylamino]-4(1H)-pyrimidone di-hydrochloride

In 30 ml of pyridine were stirred 1.93 g of 3-[m-[(3-methyl)piperidinomethyl]phenoxy]propylamine and 1.5 g pf 5-(2-(Z)-butenyl)-6-methyl-2-nitroamino)-4(1H)-pyrimidone under reflux for 3 days. After evaporating the solvent from the reaction mixture, the residue thus formed was purified by column chromatography (silica gel, methanol-chloroform), and then ethanolic hydrochloric acid was added to the purified product to form a salt thereof, which was collected and recrystallized from a mixture of ethanol and ether to provide 1.0 g of the desired product.

Melting point: 141 to 145°C

Mass spectrum (m/z): 424 ($M^+$)

Elemental analysis for $C_{25}H_{36}N_4O_2.2HCl.0.5H_2O$:

| | C | H | N | Cl |
|---|---|---|---|---|
| Calculated: | 59.28% | 7.76% | 11.06% | 14.00% |
| Found: | 59.25% | 7.89% | 11.06% | 13.88% |

(It is confirmed by $^{13}C$ NMR that trans/cis was 4.4/95.6).

Example 41

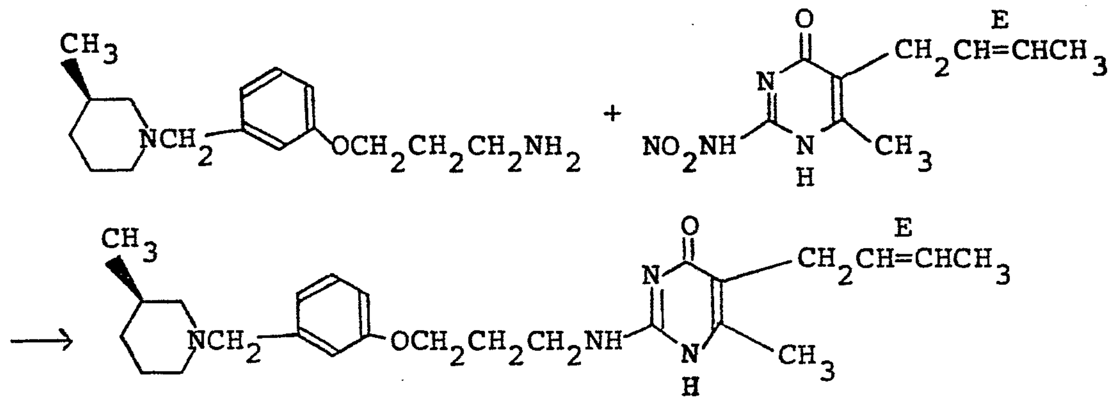

In 15 ml of pyridine were reacted 1.4 g of 3-[3-(R-(-)-3-methylpiperidinomethyl)phenoxy]propylamine and 1.2 g of 5-(2-(E)-butenyl)-6-methyl-2-nitroamino-4(1H)-pyrimidone for 24 hours at 100°C. The solvent was distilled off from the reaction mixture under reduced pressure and the residue thus formed was purified by silica gel column chromatography using a mixture of chloroform and methanol as a developing solvent and recrystallized twice from a mixture of ethanol and acetonitrile to provide 1.1 g of 5-(2-(E)-butenyl)-6-methyl-2- [3-[m-(R-(-)-3-methylpiperidinomethyl)phenoxy]-propylamino]-4(1H)-pyrimidone of melting point 97 to 98°C and showing $[\alpha]_D^{23}$ - 8.31° (C = 1.1, MeOH).

Elemental analysis for $C_{25}H_{36}N_4O_2$:

| | C | H | N |
|---|---|---|---|
| Calculated: | 70.72% | 8.55% | 13.20% |
| Found: | 70.65% | 8.62% | 13.09% |

Example 42

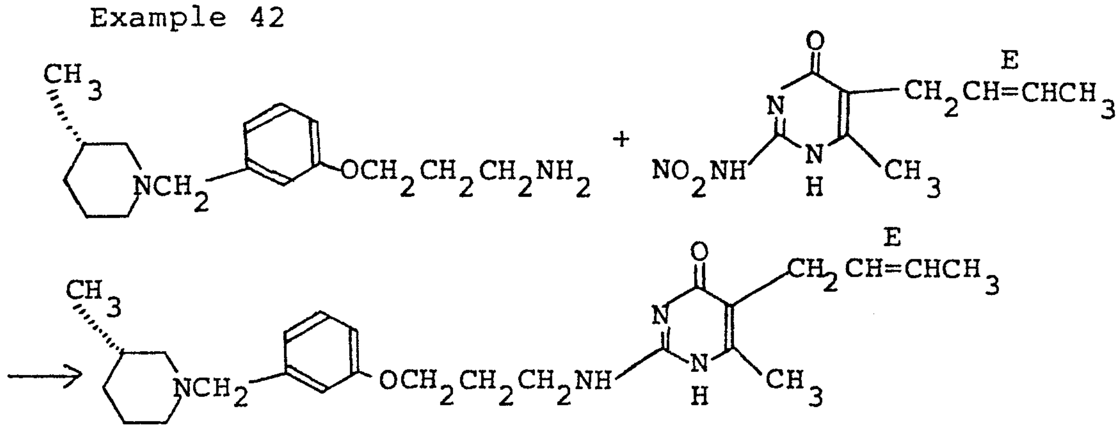

In 20 ml of pyridine were reacted 1.5 g of 3-[3-(S-(+)-3-methylpiperidinomethyl)phenoxy]propylamine and 1.2 g of 5-(2-(E)-butenyl)-6-methyl-2-nitroamino-4(1H)-pyrimidone for 24 hours at 100°C. After distilling off the solvent from the reaction mixture under reduced pressure, the residue thus formed was purified by column chromatography using a mixture of chloroform and methanol as a developing solvent and recrystallized twice from a mixture of ethanol and acetonitrile to provide 0.24 g of 5-(2-(E)-butenyl)-6-methyl-2- [3-[m-(S-(+)-3-methylpiperidino-methyl)phenoxy]propylamino]-4(1H)pyrimidone having a melting point of 98 to 99°C and showing $[\alpha]_D^{23}$ + 8.39° (C = 0.71, MeOH).

Elemental analysis for $C_{25}H_{36}N_4O_2$:

| | C | H | N |
|---|---|---|---|
| Calculated: | 70.72% | 8.55% | 13.20% |
| Found: | 70.73% | 8.56% | 13.16% |

Example 43

In 10 ml of ethanol were dissolved 2.0 g of 5-(2-(E)-butenyl)-6-methyl-2-[[3-[m-(methylpiperidinomethyl)-phenoxy]propyl]amino}-4(1H)-pyrimidone and 0.54 g of maleic acid and then after adding 25 ml of ethyl acetate to the solution, the mixture was allowed to stand for 20 hours to deposit crystals which were collected by filtration to provide 2.38 g of 5-(2-(E)-butenyl)-6-methyl-2- [3-[m-(3-methylpiperidinomethyl)phenoxy]propylamino]-4(1H)-pyrimidone maleate of m.p. 135 to 138°C.

Elemental analysis for $C_{29}H_{40}N_4O_6$:

| | C | H | N |
|---|---|---|---|
| Calculated: | 64.42% | 7.46% | 10.36% |
| Found: | 64.24% | 7.58% | 10.26% |

By following the methods above the following compounds were obtained (Examples 44 and 45):

Example 44

5-(2-(E)-Butenyl)-6-methyl-2- [3-[m-(S-(+)-3-methylpiperidinomethyl)phenoxy]propyl amino]-4(1H)-pyrimidone maleate:

Melting point: 140 to 144°C

$[\alpha]_D^{23}$ + 7.12° (C = 0.98, MeOH)

Elemental analysis for $C_{29}H_{40}N_4O_6$:

| | C | H | N |
|---|---|---|---|
| Calculated: | 64.42% | 7.46% | 10.36% |
| Found: | 64.39% | 7.64% | 10.34% |

Example 45

5-(2-(E)-Butenyl)-6-methyl-2- [3-[m-(R-(-)-3-methylpiperidinomethyl)phenoxy]propylamino]-4(1H)-pyrimidone maleate:

Melting point: 139 to 143°C

$[\alpha]_D^{23}$ - 7.18° (C = 1.20, MeOH)

Elemental analysis for $C_{29}H_{40}N_4O_6$:

| | C | H | N |
|---|---|---|---|
| Calculated: | 64.42% | 7.46% | 10.36% |
| Found: | 64.29% | 7.56% | 10.28% |

Example 46

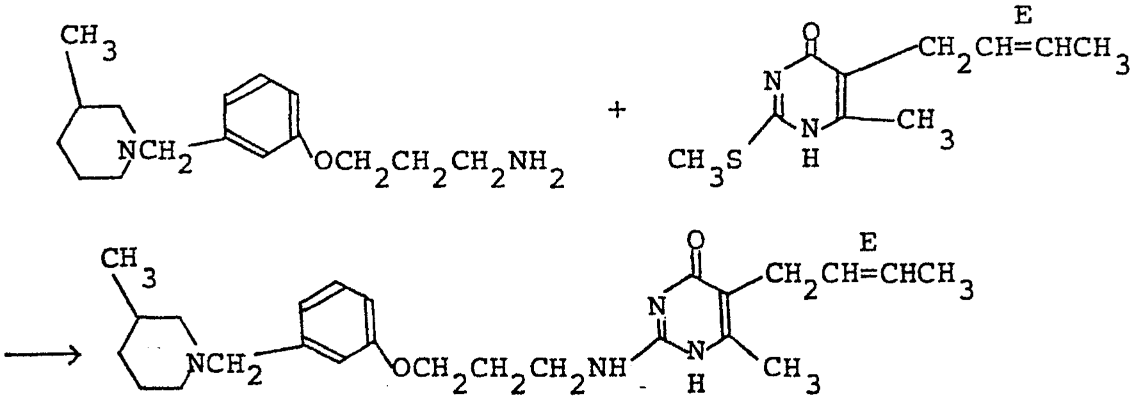

In 5 ml of toluene were refluxed 1.0 g of 3-[3-(3-methylpiperidinomethyl)phenoxy]propylamine and 0.8 g of 5-(2-(E)-butenyl)-6-methyl-2-methylthio-4(1H)-pyrimidone for 24 hours and then the solvent was distilled off under reduced pressure. To the residue thus formed was added 8 ml of acetonitrile containing 20% ethanol followed by stirring and crystals thus deposited were collected by filtration to provide 1.07 g of 5-(2-(E)-butenyl)-6-methyl-2- [3-[m-(3-methylpiperidinomethyl)-phenoxy]propylamino]-4(1H)-pyrimidone having a melt point of 98 to 99°C.

Example 47

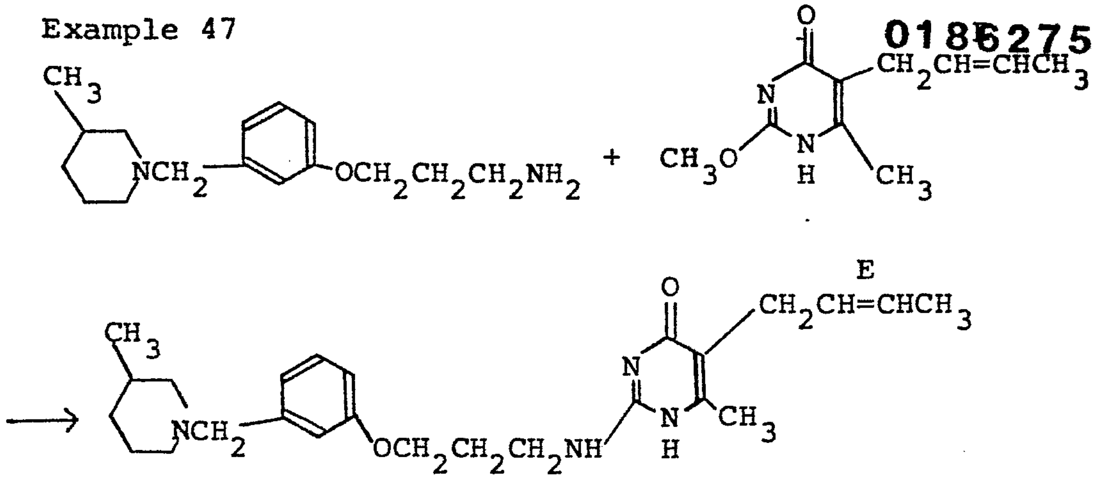

After refluxing 1.0 g of 3-[3-(3-methylpiperidinomethyl)phenoxy]propylamine and 0.74 g of 5-(2-(E)-butenyl)-6-methyl-2-methoxy-4(1H)-pyrimidone in 5 ml of toluene for 24 hours, the solvent was distilled off from the reaction mixture under reduced pressure. To the residue thus formed was added 8 ml of acetonitrile containing 20% ethanol followed by stirring and crystals thus deposited were collected by filtration to provide 0.99 g of 5-(2-(E)-butenyl)-6-methyl-2- [3-[m-(3-methylpiperidinomethyl)phenoxy]propylamino]-4(1H)-pyrimidone of melting point 98 to 99°C.

Example 48

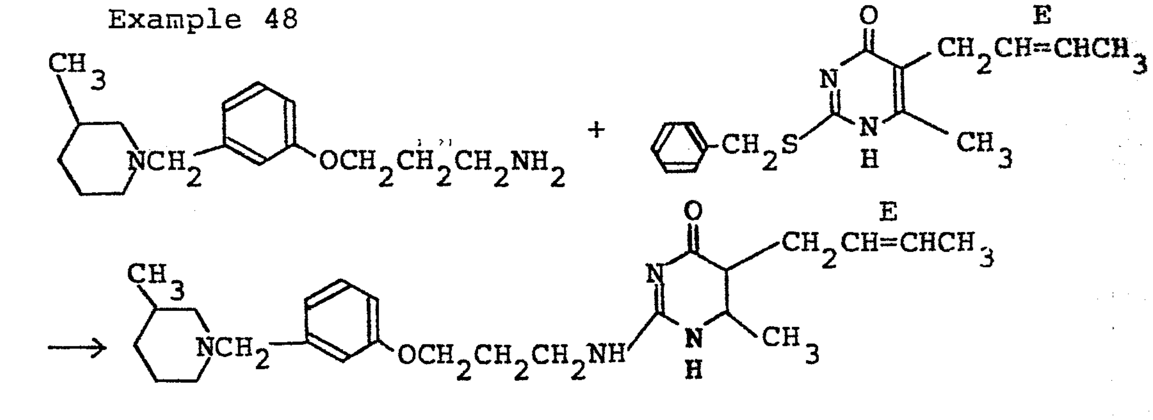

After refluxing 1.0 g of 3-[3-(3-methylpiperidinomethyl)phenoxy]propylamine and 1.09 g of 5-(2-(E)-butenyl)-6-methyl-2-benzylthio-4(1H)-pyrimidone in 5 ml of toluene for 24 hours, the solvent was distilled off from the reaction mixture under reduced pressure. To the residue thus formed was added 8 ml of acetonitrile containing 20% ethanol followed by stirring and crystals thus deposited were collected by filtration to provide 0.76 g of 5-(2-(E)-butenyl)-6-methyl-2- [3-[m-(3-methylpiperidinomethyl)phenoxy]propyl amino]-4(1H)-pyrimidone of melting point 98 to 99°C.

Example 49 (Tablet)

| | | |
|---|---|---|
| 5-(2-Butenyl)-6-methyl-2-[3-[m-(3-methylpiperidinomethyl)phenoxy]propylamino]-4(1H)-pyrimidone (hereinafter referred to as "HB-408") | | 100 mg |
| Crystalline cellulose | | 19 mg |
| Hydroxypropyl cellulose | | 3 mg |
| Calcium carboxymethyl cellulose | | 2 mg |
| Magnesium stearate | | 1 mg |
| | Total | 125 mg |

After uniformly mixing 100 g of HB-408 and 19 g of crystalline cellulose, 30 ml of a 10%(w/v) aqueous solution of hydroxypropyl cellulose was added to the mixture and the resultant mixture was granulated by a wet granulation method. The obtained granules were dried and then mixed with 2 g of calcium carboxymethyl cellulose and 1 g of magnesium stearate and the mixture was press-tableted into tablets (125 mg per tablet).

CLAIMS:

1. A 2-substituted amino-4-(1H)-pyrimidone derivative represented by the general formula (I) or an acid addition salt thereof

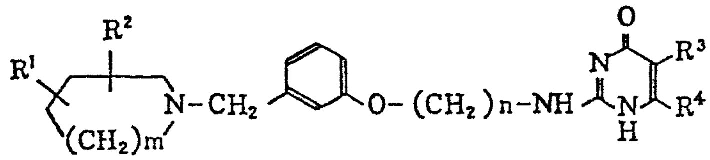

(I)

wherein $R^1$ and $R^2$ are the same or different and selected from a hydrogen atom and $C_1$ to $C_5$ alkyl groups; $R^3$ represents an alkyl, alkenyl, or alkynyl group of up to 10 carbon atoms which may be substituted by halogen atom(s), or a $C_1$ to $C_{10}$ alkyl group interrupted by an oxygen or sulfur atom; $R^4$ represents a hydrogen atom or a $C_1$ to $C_5$ alkyl group; m represents an integer of 1 to 3; and n represents an integer of 2 to 4; with the proviso that $R^4$ is a $C_1$ to $C_5$ alkyl group when $R^1$ and $R^2$ are hydrogen atoms, $R^3$ is an alkyl group, m is 2, and n is 3.

2. A compound according to claim 1 wherein $R^3$ is an alkyl or alkenyl group of up to 10 carbon atoms and $R^4$ is a $C_1$ to $C_5$ alkyl group.

3. A compound according to claim 1 which is 5-butyl-6-methyl-2-[3-[m-(piperidinomethyl)phenoxy]propylamino]-4(1H)-pyrimidone or an acid addition salt thereof; 5-(2-butenyl)-6-methyl-2-[3-[m-(3-methylpiperidinomethyl)-phenoxy]propylamino]-4(1H)-pyrimidone or an acid addition salt thereof; or 5-(2-(E)-butenyl)-6-methyl-2-[3-[m-(3-methylpiperidino-methyl)phenoxy]propylamino]-4(1H)-pyrimidone or an acid addition salt thereof.

4. A process of producing a 2-substituted amino-4(1H)-pyrimidone derivative represented by the general formula

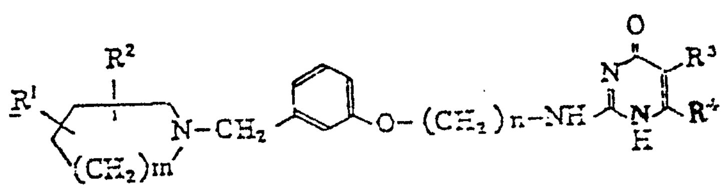

or an acid addition salt thereof

wherein $R^1$ and $R^2$ are the same or different and selected from a hydrogen atom and $C_1$ to $C_5$ alkyl groups; $R^3$ represents an alkyl, alkenyl, or alkynyl group of up to 10 carbon atoms which may be substituted by halogen atom(s), or a $C_1$ to $C_{10}$ alkyl group interrupted by an oxygen or sulfur atom; $R^4$ represents a hydrogen atom or a $C_1$ to $C_5$ alkyl group; m represents an integer of 1 to 3; and n represents an integer of 2 to 4; with the proviso that $R^4$ is a $C_1$ to $C_5$ alkyl group when $R^1$ and $R^2$ are hydrogen atoms, $R^3$ is an alkyl group, m is 2, and n is 3

which comprises either :

(a) reacting a phenoxyalkylamine represented by the general formula

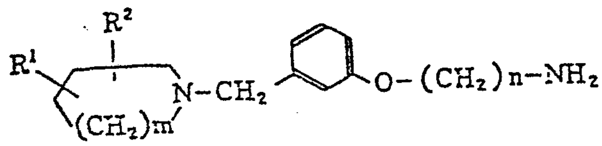

(wherein $R^1$, $R^2$, m and n are as defined above) with a 2-nitroamino-, 2-($C_1$ to $C_5$) alkylthio-, 2-($C_1$ to $C_5$) alkoxy- or 2-aralkylthio-4(H)-pyrimidone derivative represented by the general formula

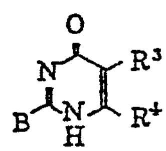

(wherein $R^3$ and $R^4$ are as defined above and B represents a nitroamino group, a $C_1$ to $C_5$ alkylthio group, a $C_1$ to $C_5$ alkoxy group or an aralkythio group); or

(b) reacting a phenoxyalkylguanidine represented by the general formula

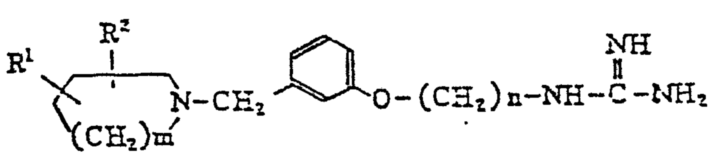

(wherein $R^1$, $R^2$, m and n are as defined above ) with

an acylacetic acid ester derivative represented by the general formula

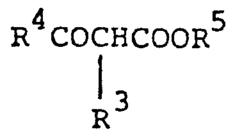

(wherein $R^3$, $R^4$ and $R^5$ are as defined above); or

(c) reacting a phenoxyalkylamine represented by the general formula

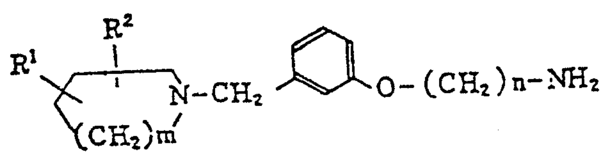

(wherein $R^1$, $R^2$, m and n are as defined above) with a 2-halogeno-4(1H)-pyrimidone derivative represented by the general formula

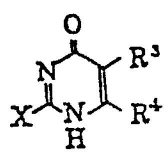

(wherein $R^3$ and $R^4$ are as defined above and X represents a halogen atom); or

(d) reacting a halogen compound represented by the general formula

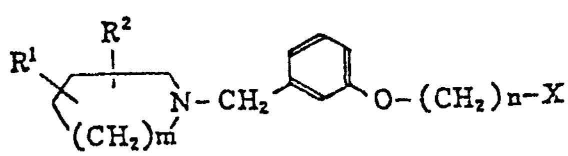

(wherein $R^1$, $R^2$, m, n and X are as defined above) with a 2-amino-4(1H)—pyrimidone derivative represented by the general formula

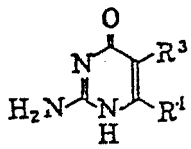

(wherein $R^3$ and $R^4$ are as defined above);or

(e) reacting a phenol derivative represented by the general formula

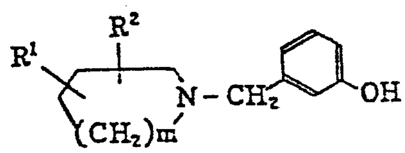

(wherein $R^1$, $R^2$ and m are as defined above) with a pyrimidone derivative represented by the general formula

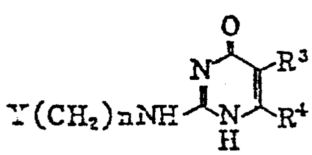

(wherein, $R^3$, $R^4$ and n are as defined above and Y represents a halogen atom or organic sulfonyloxy

group) in the presence of alkali; and optionally subjecting the product of a), b), c), d) or e) to a salt formation step.

5. A process according to claim 4 wherein $R^3$ is an alkyl or alkenyl group of up to 10 carbon atoms and $R^4$ is a $C_1$ to $C_5$ alkyl group.

6. A process according to claim 5 wherein m is 1 or 2 and n is 3.

7. A process according to claim 4 wherein $R^1$ is methyl (3-position), $R^2$ is hydrogen, $R^3$ is 2-butenyl, $R^4$ is methyl, m is 2 and n is 3; or $R^1$ and $R^2$ are both hydrogen atoms, $R^3$ is butyl, $R^4$ is methyl, m is 2 and n is 3; or $R^1$ is methyl (3-position), $R^2$ is hydrogen, $R^3$ is 2-(E)-butenyl, $R^4$ is methyl, m is 2 and n is 3.

8. A medical composition containing a compound according to any one of claims 1 to 3.